# EUROPEAN PATENT SPECIFICATION

(11) **EP 1 877 556 B1**
(45) Date of publication and mention of the grant of the patent: **14.09.2011**
(21) Application number: 06739567.3
(22) Date of filing: 24.03.2006
(51) Int. Cl.: C12N 15/11, A61K 31/7105, A61P 23/00

(54) **USE OF ANTI-TNF OR ANTI-IL1 RNAI TO SUPPRESS PRO- INFLAMMATORY CYTOKINE ACTIONS LOCALLY TO TREAT PAIN**
VERWENDUNG VON ANTI-TNF ODER ANTI-IL1-RNAI ZUR UNTERDRÜCKUNG DER WIRKUNG ENTZÜNDUNGSFÖRDERNDER CYTOKINE ZUR LOKALEN SCHMERZBEHANDLUNG
UTILISATION DE RNAI ANTI-TNF OU ANTI-IL-1 POUR SUPPRIMER LOCALEMENT L'ACTIVITE DE CYTOKINES PRO-INFLAMMATOIRES AFIN DE TRAITER LA DOULEUR

(30) Priority: 25.03.2005 US 665481 P
(43) Date of publication of application: 16.01.2008
(73) Proprietor: Medtronic, Inc., Minneapolis, MN 55432-5604 (US)
(72) Inventor: BURRIGHT, Eric, N., Eagan, MN 55123 (US); PADUA, Rodolfo, Richfield, MN 55423 (US); SCHROEDER, Peter, T., Minneapolis, MN 55418 (US); CHRISTIANSON, Jennifer, M., Princeton, MN 55082 (US); SHAFER, Lisa, L., Stillwater, MN 55082 (US)
(74) Representative: Green, Mark Charles
(86) International application number: PCT/US2006/010853
(87) International publication number: WO 2006/104913

(56) References cited:
- WO-A-03/070897
- WO-A-2004/100987
- WO-A-2005/039393
- SORENSEN D R ET AL: "Gene Silencing by Systemic Delivery of Synthetic siRNAs in Adult Mice" JOURNAL OF MOLECULAR BIOLOGY, LONDON, GB, vol. 327, no. 4, 4 April 2003 (2003-04-04), pages 761-766, XP004454177 ISSN: 0022-2836

### SEQUENCE LISTING

<110> Medtronic Inc.
   Burright, Eric
   Padua, Rudy
   Schroeder, Peter
   Christianson, Jennifer
   shafer, Lisa
<120> Methods and sequences to Suppress Pro-Inflammatory cytokine
   Actions Locally to Treat Pain
<130> PA0021263.00
<150> 10/972,157
   <151> 2004-10-22
<150> 60/665,481
   <151> 2005-03-25
<160> 49
<170> PatentIn version 3.3
<210> 1
   <211> 19
   <212> DNA
   <213> Rattus sp.
<400> 1
   gaatgacctg ttctttgag 19
<210> 2
   <211> 19
   <212> DNA
   <213> Rattus sp.
<400> 2
   tgaaagctct ccacctcaa 19
<210> 3
   <211> 19
   <212> DNA
   <213> Rattus sp.
<400> 3
   ggtacatcag cacctctca 19
<210> 4
   <211> 19
   <212> DNA
   <213> Rattus sp.
<400> 4
   cagttcccca actggtaca 19
<210> 5
   <211> 19
   <212> DNA
   <213> Rattus sp.
<400> 5
   agatagaagt caagaccaa 19
<210> 6
   <211> 19
   <212> DNA
   <213> Rattus sp.
<400> 6
   taagccaaca agtggtatt 19
<210> 7
   <211> 21
   <212> DNA
   <213> Artificial
<220>
   <223> Control nucleic acid sequence
<400> 7
   aagactgtgg ctacaacatt c 21
<210> 8
   <211> 19
   <212> DNA
   <213> Rattus sp.
<400> 8
   aagcatgatc cgagatgtg 19
<210> 9
   <211> 19
   <212> DNA
   <213> Rattus sp.
<400> 9
   catgatccga gatgtggaa 19
<210> 10
   <211> 19
   <212> DNA
   <213> Rattus sp.
<400> 10
   accacgctct tctgtctac 19
<210> 11
   <211> 19
   <212> DNA
   <213> Rattus sp.
<400> 11
   ccacgctctt ctgtctact 19
<210> 12
   <211> 19
   <212> DNA
   <213> Rattus sp.
<400> 12
   cacgctcttc tgtctactg 19
<210> 13
<211> 19
   <212> DNA
   <213> Rattus sp.
<400> 13
   acgctcttct gtctactga 19
<210> 14
   <211> 19
   <212> DNA
   <213> Homo sapiens
<400> 14
   gtgaaatgat ggcttatta 19
<210> 15
   <211> 19
   <212> DNA
   <213> Homo sapiens
<400> 15
   cgatgcacct gtacgatca 19
<210> 16
   <211> 19
   <212> DNA
   <213> Homo sapiens
<400> 16
   taactgactt caccatgca 19
<210> 17
   <211> 19
   <212> DNA
   <213> Homo sapiens
<400> 17
   gaacctatct tcttcgaca 19
<210> 18
   <211> 19
   <212> DNA
   <213> Homo sapiens
<400> 18
   ccagggacct ctctctaat 19
<210> 19
   <211> 19
   <212> DNA
   <213> Homo sapiens
<400> 19
   agggacctct ctctaatca 19
<210> 20
   <211> 19
   <212> DNA
   <213> Homo sapiens
<400> 20
gcctgtagcc catgttgta 19
<210> 21
   <211> 19
   <212> DNA
<213> Homo sapiens
<400> 21
   tgtagcccat gttgtagca 19
<210> 22
   <211> 22
   <212> DNA
   <213> Artificial
<220>
   <223> control nucleic acid sequence
<400> 22
   tgacacagcc gctactacat tg 22
<210> 23
   <211> 19
   <212> DNA
   <213> Homo sapiens
<400> 23
   acattactat tgcgtggta 19
<210> 24
   <211> 19
   <212> DNA
   <213> Homo sapiens
<400> 24
   gaatgagcct aacttatgt 19
<210> 25
   <211> 19
   <212> DNA
   <213> Homo sapiens
<400> 25
   ataggctcat cgtgatgaa 19
<210> 26
   <211> 19
   <212> DNA
   <213> Homo sapiens
<400> 26
   gaactatact tgtcatgca 19
<210> 27
   <211> 19
   <212> DNA
   <213> Homo sapiens
<400> 27
   cgacggtcac cttcatcta 19
<210> 28
   <211> 19
   <212> DNA
   <213> Homo sapiens
<400> 28
   acggtcacct tcatctaaa 19
<210> 29
   <211> 19
   <212> DNA
   <213> Homo sapiens
<400> 29 19
   acctacgtct gcactacta 19
<210> 30
   <211> 19
   <212> DNA
   <213> Homo sapiens
<400> 30 19
   tcctgccgtt catctcata 19
<210> 31
   <211> 19
   <212> DNA
   <213> Homo sapiens
<400> 31 19
   ccactcactt actcgtaca 19
<210> 32
   <211> 19
   <212> DNA
   <213> Homo sapiens
<400> 32 19
   acattgaagt gccattgat 19
<210> 33
   <211> 19
   <212> DNA
   <213> Homo sapiens
<400> 33 19
   acgtctgcac tactagaaa 19
<210> 34
   <211> 19
   <212> DNA
   <213> Homo sapiens
<400> 34 19
   ctactagaaa tgcttctta 19
<210> 35
   <211> 19
   <212> DNA
   <213> Homo sapiens
<400> 35 19
   cggcattatt ggagtgaaa 19
<210> 36
   <211> 19
   <212> DNA
   <213> Homo sapiens
<400> 36
   acaaaggaac ctacttgta 19
<210> 37
   <211> 19
   <212> DNA
   <213> Homo sapiens
<400> 37
   aaccagtacc ggcattatt 19
<210> 38
   <211> 19
   <212> DNA
   <213> Homo sapiens
<400> 38
   aattcgattt gctgtacca 19
<210> 39
   <211> 19
   <212> DNA
   <213> Homo sapiens
<400> 39
   tttaatgtat cgctaccaa 19
<210> 40
   <211> 19
   <212> DNA
   <213> Homo sapiens
<400> 40
   gagcttgaag gaactacta 19
<210> 41
   <211> 19
   <212> DNA
   <213> Homo sapiens
<400> 41
   acatgccggc tcagagaat 19
<210> 42
   <211> 19
   <212> DNA
   <213> Homo sapiens
<400> 42
   gccggctcag agaatacta 19
<210> 43
   <211> 19
   <212> DNA
   <213> Homo sapiens
<400> 43
   catcagacgt ggtgtgcaa 19
<210> 44
   <211> 19
   <212> DNA
   <213> Homo sapiens
<400> 44
   ctcacttgcc tgccgataa 19
<210> 45
   <211> 19
   <212> DNA
   <213> Homo sapiens
<400> 45
   tcacttgcct gccgataag 19
<210> 46
   <211> 19
   <212> DNA
   <213> Homo sapiens
<400> 46
   acacgacttc atccacgga 19
<210> 47
   <211> 55
   <212> DNA
   <213> Artificial
<220>
   <223> short hairpin nucleic acid of consensus rat/human IL-2beta
   sequence.
<400> 47
   ccagttcccc aactggtaca ttcaagagat gtaccagttg gggaactgtt ttttg 55
<210> 48
   <211> 54
   <212> DNA
   <213> Rattus sp.
<400> 48
   acgctcttct gtctactgat tcaagagatc agtagacaga agagcgtttt tttg 54
<210> 49
   <211> 60
   <212> DNA
   <213> Artificial
<220>
   <223> control short hairpin nucleic acid sequence
<400> 49
   tgacacagcc gctactacat tgttcaagag acaatgtagt agcggctgtg tcattttttg 60

## Description

### FIELD OF THE INVENTION

The present invention relates to delivering inhibitory nucleic acid molecules locally to the perispinal region or intrathecal space of the spinal cord to locally and specifically suppress expression of the pro-inflammatory cytokines interleukin-1 beta and tumor necrosis factor alpha and their receptors to alleviate acute or neuropathic pain.

### BACKGROUND OF THE INVENTION

Pain can be divided into two types: acute pain and neuropathic pain. Acute pain refers to pain experienced when tissue is being damaged or is damaged. Acute pain serves at least two physiologically advantageous purposes. First, it warns of dangerous environmental stimuli (such as hot or sharp objects) by triggering reflexive responses that end contact with the dangerous stimuli. Second, if reflexive responses do not avoid dangerous environmental stimuli effectively, or tissue injury or infection otherwise results, acute pain facilitates recuperative behaviors. For example, acute pain associated with an injury or infection encourages an organism to protect the compromised area from further insult or use while the injury or infection heals. Once the dangerous environmental stimulus is removed, or the injury or infection has resolved, acute pain, having served its physiological purpose, ends.

As contrasted to acute pain, neuropathic pain serves no beneficial purpose. Neuropathic pain results when pain associated with an injury or infection continues in an area once the injury or infection has resolved. The biological basis for this type of pain that exists absent physical injury or infection baffled scientists for many years. Recently, however, evidence has mounted that neuropathic pain is caused, at least in part, by on-going (and unneeded) activation of the immune system after an injury or infection has healed. See, for example, WATKINS & MAIER (2004), PAIN, CLINICAL UPDATES, 1-4.

Local immune system activation begins when damaged cells secrete signals that recruit immune system cells to the area. One type of recruited immune system cell is the macrophage. Macrophages release interleukin-1 beta ("IL-1β") and tumor necrosis factor alpha ("TNFα"), pro-inflammatory cytokines heavily involved in orchestrating the immediate and local physiological effects of injury or infection. For instance, once released, pro-inflammatory cytokines promote inflammation (swelling and redness caused by increased blood flow to the area which delivers recruited immune system cells more quickly) and also increase sensitivity to pain (by increasing the excitability and transmission of sensory nerves carrying pain information to the brain). Thus, pro-inflammatory cytokines are involved in the beneficial physiological and recuperative effects of acute pain.

Normally after an injury or infection heals, the local immune system response ceases, inflammation recedes and the increased sensitivity to pain abates. In some individuals, however, signals that terminate the immune system response are not effective entirely and pro-inflammatory cytokine activity in the area remains active. In these individuals, sensory nerves carrying pain information to the brain remain sensitized in the absence of injury or infection and the individuals can experience neuropathic pain.

Sciatica provides an example of pain that can transition from acute to neuropathic pain. Sciatica refers to pain associated with the sciatic nerve which runs from the lower part of the spinal cord (the lumbar region), down the back of the leg and to the foot. Sciatica generally begins with a herniated disc. The herniated disc itself leads to local immune system activation. The herniated disc also may damage the nerve root by pinching or compressing it, leading to additional immune system activation in the area. In most individuals, the acute pain and immune system activation associated with the injury cease once the damage has been repaired. In those individuals where immune system activation does not abate completely, however, neuropathic pain may result.

As the foregoing suggests, inhibiting the actions of pro-inflammatory cytokines can provide an effective strategy for treating acute and neuropathic pain. Inhibiting the immune system, however, is problematic as a general treatment because it leaves an individual vulnerable to infection and unable to repair tissue injuries effectively. Thus, treatments that inhibit pro-inflammatory cytokines throughout the body generally are not appropriate except in the most extreme cases of neuropathic pain. Other pain treatments likewise are not effective or appropriate for treating acute or neuropathic pain caused by pro-inflammatory cytokines. For example, narcotics do not treat pain mediated by the pro-inflammatory cytokines because narcotics block opiate receptors, a receptor type not directly involved in many effects of the pro-inflammatory cytokines. A need exists, therefore, for a locally-administered pain treatment that suppresses the actions of the pro-inflammatory cytokines.

Recent developments in genetic technologies have made the selective suppression of certain proteins' actions (such as the pro-inflammatory cytokines) possible. Some background in the art is required to understand the potential impact of these technologies. Generally, for a protein to exert an effect, the cell that will use or secrete the protein must create It. To create a protein the cell first makes a copy of the protein's gene sequence in the nucleus of the call. This copy of the gene sequence that encodes for the protein (called messenger RNA ("mRNA)) leaves the nucleus and is trafficked to a region of the cell containing ribosomes. Ribosomes read the sequence of the mRNA and create the protein for which it encodes. This process of new protein synthesis is known as translation. A variety of factors affect the rate and efficiency of protein translation. Among the most significant of these factors Is the intrinsic stability of the mRNA Itself. If the mRNA is degraded quickly within the cell (such as before it reaches a ribosome). It is unable to serve as a template for new protein translation, thus reducing the cell's ability to create the protein for which it encoded.

Based on the foregoing, the technology of RNA interference ("RNAi") has emerged. RNAi is, in fact, a naturally-occurring mechanism for suppressing gene expression and subsequent protein translation. RNAi suppresses protein translation by either degrading the mRNA before it can be translated or by binding the mRNA and directly preventing its translation. This technology provides an avenue to suppress the expression and actions of pro-inflammatory cytokines and their receptors within a given population of cells (i.e. locally) rather than globally inhibiting the immune system in an attempt to treat acute and neuropathic pain.
[0010a] WO 2005/039393 of Medtronic inc., published on 6 May 2005, describes the use of TNF modifying agents for treating a CNS disorder, such agents being locally administered. The TNF modifying agent may be siRNA targetted to TNF-alpha.

### SUMMARY OF THE INVENTION

The present invention describe methods and nucleic acid molecules for using RNA Interference ("RNAi") to locally suppress expression of both pro-inflammatory cytokines and/or their receptors to treat acute or neuropathic pain. Specifically, RNAi is mediated by double stranded RNA ("dsRNA"), short hairpin RNA ("shRNA") or other nucleic add molecules with similar characteristics. These nucleic acid molecules are processed or cut into smaller pieces by cellular enzymes including Dicer and Drosha. The smaller fragments of the nucleic acid molecules can then be taken up by a protein complex (the RISC complex) that mediates degradation of mRNAs. The RISC complex will degrade mRNA that complementarily base pairs with the nucleic acid molecules it has taken up. In this manner the mRNA is specifically destroyed, thus preventing the encoded-for protein from being made.

The understanding of the mechanism of RNAi now allows geneticists to create nucleic acid molecules with sequences that are homologous to known gene sequences in order to suppress the expression or formation of certain proteins within a cell. In this invention, nucleic add molecules that are homologous to pro-inflammatory cytokine mRNA sequences and/or pro-inflammatory cytokine receptor mRNA sequences, are introduced into cells locally to suppress expression of these proteins. These nucleic add molecules specifically suppress the expression of amino add sequences that encode for (interleukin-1 beta ("IL-1β"). tumor necrosis factor alpha ("TNFα"), the type I IL-1β receptor, the type II IL-1β receptor, the type I TNFα receptor and the type II TNFα receptor. Suppressing expression of these proteins locally treats pain in an area without globally inhibiting the Immune system.

The present invention relates to the use of nucleic acid molecules in the manufacture of a medicament for the treatment of pain in accordance with the claims attached. In one embodiment of the present invention, the inflammatory response proteins are pro-inflammatory cytokines. In another embodiment, of the methods of the present invention, the nucleic acid molecules comprise mammalian nucleotide sequences that suppress the expression of amino acid sequences encoding for interleukin-1 beta ("IL-1β"). In another embodiment of the present invention, the nucleic acid molecules comprise mammalian nucleotide sequences that comprise SEQ ID NO: 14. In another embodiment of the methods of the present invention, the nucleic acid molecules comprise mammalian nucleotide sequences that comprise SEQ ID NO: 15. In another embodiment of the present invention, the nucleic acid molecules comprise mammalian nucleotide sequences that comprise SEQ ID NO: 16. In another embodiment of the present invention, the nucleic acid molecules comprise mammalian nucleotide sequences that comprise SEQ ID NO: 17. In another embodiment of the present invention, the nucleic add molecules comprise mammalian nucleotide sequences that comprise SEQ ID NO: 14 In shNA format. In another embodiment of the present invention, the nucleic acid molecules comprise mammalian nucleotide sequences that comprise SEQ ID NO: 15 in shNA format. In another embodiment of the present invention, the nucleic acid molecules comprise mammalian nucleotide sequences that comprise SEQ ID NO: 16 in shNA format in another embodiment of the present invention, the nucleic acid molecules comprise mammalian nucleotide sequences that comprise SEQ ID NO: 17 in shNA format. In another embodiment of the present invention, the nucleic acid molecules comprise mammalian nucleotide sequences that comprise combinations of SEQ ID NO: 14; SEQ ID NO: 15; SEQ ID NO: 16; and SEQ ID NO: 17 In short interfering nucleic acid ("siNA") or short hairpin nucleic acid ("shNAs") formats.

In one embodiment of the present invention, the nucleic acid molecules comprise mammalian nucleotide sequences that suppress the expression of amino add sequences encoding for tumor necrosis factor alpha ("TNFα"). In another embodiment of the present invention, the nucleic add molecule comprises mammalian nucleotide sequences that comprise SEQ ID NO: 18. In another embodiment of the present invention, the nucleic acid molecules comprise mammalian nucleotide sequences that comprise SEQ ID NO: 19. In another embodiment of the present invention, the nucleic acid molecules comprise mammalian nucleotide sequences that comprise SEQ ID NO: 20. In another embodiment of the present invention, the nucleic acid molecules comprise mammalian nucleotide sequences that comprise SEQ ID NO: 21. In another embodiment of the present invention, the nucleic acid molecules comprise mammalian nucleotide sequences that comprise SEQ ID NO: 18 in shNA format. In another embodiment of the present invention, the nucleic acid molecules comprise mammalian nucleotide sequences that comprise SEQ ID NO: 19 in shNA format. In another embodiment of the present invention, the nucleic acid molecules comprise mammalian nucleotide sequences that comprise SEQ ID NO: 20 in shNA format. In another embodiment of the present invention, the nucleic acid molecules comprise mammalian nucleotide sequences that comprise SEQ ID NO: 21 in shNA format. In another embodiment of the present invention, the nucleic acid molecules comprise mammalian nucleotide sequences that comprise combinations of SEQ ID NO: 18; SEQ ID NO: 19; SEQ ID NO: 20; and SEQ ID NO: 21 in siNA or shNA formats. In another embodiment of the present invention, the nucleic acid molecules comprise any combination of nucleic acid molecules that suppress the expression of amino acid sequences encoding for IL-1β and nucleic acid molecules that suppress the expression of amino acid sequences encoding for TNFα.

In one embodiment of the present invention, the inflammatory response proteins are pro-inflammatory cytokine receptors. In another embodiment of the present invention, the pro-inflammatory cytokine receptors are type I IL-1β receptors. In another embodiment of the present invention, the nucleic acid molecules comprise mammalian nucleotide sequences that comprise SEQ ID NO: 23. In another embodiment of the present invention, the nucleic acid molecules comprise mammalian nucleotide sequences that comprise SEQ ID NO: 24. In another embodiment of the present invention, the nucleic acid molecules comprise mammalian nucleotide sequences that comprise SEQ ID NO: 25. In another embodiment of the present invention, the nucleic acid molecules comprise mammalian nucleotide sequences that comprise SEQ ID NO: 26. In another embodiment of the present invention, the nucleic acid molecules comprise mammalian nucleotide sequences that comprise SEQ ID NO: 27. In another embodiment of the present invention, the nucleic acid molecules comprise mammalian nucleotide sequences that comprise SEQ ID NO: 28. In another embodiment of the present invention, the nucleic acid molecules comprise mammalian nucleotide sequences that comprise SEQ ID NO: 23 in shNA format. In another embodiment of the present invention, the nucleic acid molecules comprise mammalian nucleotide sequences that comprise SEQ ID NO: 24 in shNA format. In another embodiment of the present invention, the nucleic acid molecules comprise mammalian nucleotide sequences that comprise SEQ ID NO: 25 in shNA format. In another embodiment of the present invention, the nucleic acid molecules comprise mammalian nucleotide sequences that comprise SEQ ID NO: 26 in shNA format. In another embodiment of the present invention, the nucleic acid molecules comprise mammalian nucleotide sequences that comprise SEQ ID NO: 27 in shNA format. In another embodiment of the present invention, the nucleic acid molecules comprise mammalian nucleotide sequences that comprise SEQ ID NO: 28 in shNA format. In another embodiment of the present invention, the nucleic acid molecules comprise mammalian nucleotide sequences that comprise combinations of SEQ ID NO: 23; SEQ ID NO: 24; SEQ ID NO: 25; SEQ ID NO: 26; SEQ ID NO: 27 and SEQ ID NO: 28 in siNA or shNA formats.

In one embodiment of the present invention, the pro-inflammatory cytokine receptors are type II IL-1β receptors. In another embodiment of the present invention, the nucleic acid molecules comprise mammalian nucleotide sequences that comprise SEQ ID NO: 29. In another embodiment of the present invention, the nucleic acid molecules comprise mammalian nucleotide sequences that comprise SEQ ID NO: 30. In another embodiment of the present invention, the nucleic acid molecules comprise mammalian nucleotide sequences that comprise SEQ ID NO: 31. In another embodiment of the present invention, the nucleic acid molecules comprise mammalian nucleotide sequences that comprise SEQ ID NO: 32. In another embodiment of the present invention, the nucleic acid molecules comprise mammalian nucleotide sequences that comprise SEQ ID NO: 33. In another embodiment of the present invention, the nucleic acid molecules comprise mammalian nucleotide sequences that comprise SEQ ID NO: 34. In another embodiment of the present invention, the nucleic acid molecules comprise mammalian nucleotide sequences that comprise SEQ ID NO: 29 in shNA format. In another embodiment of the present invention, the nucleic acid molecules comprise mammalian nucleotide sequences that comprise SEQ ID NO: 30 in shNA format. In another embodiment of the present invention, the nucleic acid molecules comprise mammalian nucleotide sequences that comprise SEQ ID NO: 31 in shNA format. In another embodiment of the present invention, the nucleic acid molecules comprise mammalian nucleotide sequences that comprise SEQ ID NO: 32 in shNA format. In another embodiment of the present invention, the nucleic acid molecules comprise mammalian nucleotide sequences that comprise SEQ ID NO: 33 in shNA format. In another embodiment the present invention, the nucleic acid molecules comprise mammalian nucleotide sequences that comprise SEQ ID NO: 34 in shNA format. In another embodiment of the present invention, the nucleic acid molecules comprise mammalian nucleotide sequences that comprise combinations of SEQ ID NO: 29; SEQ ID NO: 30; SEQ ID NO: 31; SEQ.ID NO: 32; SEQ ID NO: 33 and SEQ ID NO: 34 in siNA or shNA formats.

In one embodiment of the present invention, the pro-inflammatory cytokine receptors are type I TNFα receptors. In another embodiment of the present invention, the nucleic acid molecules comprise mammalian nucleotide sequences that comprise SEQ ID NO: 35. In another embodiment of the present invention, the nucleic acid molecules comprise mammalian nucleotide sequences that comprise SEQ ID NO: 36. In another embodiment of the present invention, the nucleic acid molecules comprise mammalian nucleotide sequences that comprise SEQ ID NO: 37. In another embodiment of the present invention, the nucleic acid molecules comprise mammalian nucleotide sequences that comprise SEQ ID NO: 38. In another embodiment of the present invention, the nucleic acid molecules comprise mammalian nucleotide sequences that comprise SEQ ID NO: 39. In another embodiment of the present invention, the nucleic acid molecules comprise mammalian nucleotide sequences that comprise SEQ ID NO: 40. In another embodiment of the present invention, the nucleic acid molecules comprise mammalian nucleotide sequences that comprise SEQ ID NO: 35 in shNA format. In another embodiment of the present invention, the nucleic acid molecules comprise mammalian nucleotide sequences that comprise SEQ ID NO: 36 in shNA format. In another embodiment of the present invention, the nucleic acid molecules comprise mammalian nucleotide sequences that comprise SEQ ID NO: 37 in shNA format. In another embodiment of the present invention, the nucleic acid molecules comprise mammalian nucleotide sequences that comprise SEQ ID NO: 38 in shNA format. In another embodiment of the present invention, the nucleic acid molecules comprise mammalian nucleotide sequences that comprise SEQ ID NO: 39 in shNA format. In another embodiment of the present invention, the nucleic acid molecules comprise mammalian nucleotide sequences that comprise SEQ ID NO: 40 in shNA format. In another embodiment of the present invention, the nucleic acid molecules comprise mammalian nucleotide sequences that comprise combinations of SEQ ID NO: 35; SEQ ID NO: 36; SEQ ID NO: 37; SEQ ID NO: 38; SEQ ID NO: 39 and SEQ ID NO: 40 in siNA or shNA formats.

In one embodiment of the present invention, the pro-inflammatory cytokine receptors are type II TNFα receptors. In another embodiment of the present invention, the nucleic acid molecules comprise mammalian nucleotide sequences that comprise SEQ ID NO: 41. In another embodiment of the present invention, the nucleic acid molecules comprise mammalian nucleotide sequences that comprise SEQ ID NO: 42. In another embodiment of the present invention, the nucleic acid molecules comprise mammalian nucleotide sequences that comprise SEQ ID NO: 43. In another embodiment of the methods of the present invention, the nucleic acid molecules comprise mammalian nucleotide sequences that comprise SEQ ID NO: 44. In another embodiment of the methods of the present invention, the nucleic acid molecules comprise mammalian nucleotide sequences that comprise SEQ ID NO: 45. In another embodiment of the present invention, the nucleic acid molecules comprise mammalian nucleotide sequences that comprise SEQ ID NO: 46. In another embodiment of the present invention, the nucleic acid molecules comprise mammalian nucleotide sequences that comprise SEQ ID NO: 41 in shNA format. In another embodiment of the present invention, the nucleic acid molecules comprise mammalian nucleotide sequences that comprise SEQ ID NO: 42 in shNA format. In another embodiment of the present invention, the nucleic acid molecules comprise mammalian nucleotide sequences that comprise SEQ ID NO: 43 in shNA format. In another embodiment of the present invention, the nucleic acid molecules comprise mammalian nucleotide sequences that comprise SEQ ID NO: 44 in shNA format. In another embodiment of the present invention, the nucleic acid molecules comprise mammalian nucleotide sequences that comprise SEQ ID NO: 45 in shNA format. In another embodiment of the present invention, the nucleic acid molecules comprise mammalian nucleotide sequences that comprise SEQ ID NO: 46 in shNA format. In another embodiment of the present invention, the nucleic acid molecules comprise mammalian nucleotide sequences that comprise combinations of SEQ ID NO: 41; SEQ ID NO: 42; SEQ ID NO: 43; SEQ ID NO: 44; SEQ ID NO: 45 and SEQ ID NO: 46 in siNA or shNA formats.

In one embodiment of the present invention, the methods are used to treat acute pain. In another embodiment of the present invention, the methods are used to treat neuropathic pain. In another embodiment of the present invention, the methods are used to treat sciatica. In another embodiment of the present invention, the methods are used to treat sciatica associated with a herniated disc and resulting nerve root compression or damage.

In one embodiment of the present invention, the nucleic acid molecules are administered locally to the perispinal region. In another embodiment of the present invention, the nucleic acid molecules are administered locally to the intrathecal space of the spinal cord. In another embodiment of the present invention, the nucleic acid molecules are administered in the perispinal region or in the intrathecal space of the lumbar region of the spinal cord. In another embodiment of the present invention, the nucleic acid molecules are administered locally via a catheter and drug pump. In another embodiment of the present invention, the nucleic acid molecules are administered locally via one or more local injections. In another embodiment of the present invention, the nucleic acid molecules are administered locally via polymer release. In another embodiment of the present invention, the nucleic acid molecules are administered locally via any combination of the local administration routes of a catheter and drug pump, one or more local injections and/or polymer release.

A further embodiment of the present invention includes a nucleic acid molecules that are used to treat pain locally that comprise nucleotide sequences that suppress the expression of amino acid sequences encoding for inflammatory response proteins. In another embodiment of the nucleic acid molecules of the present invention, the nucleotide sequences are mammalian sequences that suppress the expression of amino acid sequences encoding for interleukin-1 beta ("IL-1β"). In another embodiment of the nucleic acid molecules of the present invention, the nucleotide sequence is a mammalian sequence that comprises SEQ ID NO: 14. In another embodiment of the nucleic acid molecules of the present invention, the nucleotide sequence is a mammalian sequence that comprises SEQ ID NO: 15. In another embodiment of the nucleic acid molecules of the present invention, the nucleotide sequence is a mammalian sequence that comprises SEQ ID NO: 16. In another embodiment of the nucleic acid molecules of the present invention, the nucleotide sequence is a mammalian sequence that comprises SEQ ID NO: 17. In another embodiment of the nucleic acid molecules of the present invention, the nucleotide sequence is a mammalian sequence that comprises SEQ ID NO: 14 in shNA format. In another embodiment of the nucleic acid molecules of the present invention, the nucleotide sequence is a mammalian sequence that comprises SEQ ID NO: 15 in shNA format. In another embodiment of the nucleic acid molecules of the present invention, the nucleotide sequence is a mammalian sequence that comprises SEQ ID NO: 16 in shNA format. In another embodiment of the nucleic acid molecules of the present invention, the nucleotide sequence is a mammalian sequence that comprises SEQ ID NO: 17 in shNA format.

In one embodiment of the nucleic acid molecules of the present invention, the nucleotide sequences are mammalian sequences that suppress the expression of amino acid sequences encoding for tumor necrosis factor alpha ("TNFa"). In another embodiment of the nucleic acid molecules of the present invention, the nucleotide sequence is a mammalian sequence that comprises SEQ ID NO: 18. In another embodiment of the nucleic acid molecules of the present invention, the nucleotide sequence is a mammalian sequence that comprises SEQ ID NO: 19. In another embodiment of the nucleic acid molecules of the present invention, the nucleotide sequence is a mammalian sequence that comprises SEQ ID NO: 20. In another embodiment of the nucleic acid molecules of the present invention, the nucleotide sequence is a mammalian sequence that comprises SEQ ID NO: 21. In another embodiment of the nucleic acid molecules of the present invention, the nucleotide sequence is a mammalian sequence that comprises SEQ ID NO: 18 in shNA format. In another embodiment of the nucleic acid molecules of the present invention, the nucleotide sequence is a mammalian sequence that comprises SEQ ID NO: 19 in shNA format. In another embodiment of the nucleic acid molecules of the present invention, the nucleotide sequence is a mammalian sequence that comprises SEQ ID NO: 20 in shNA format. In another embodiment of the nucleic acid molecules of the present invention, the nucleotide sequence is a mammalian sequence that comprises SEQ ID NO: 21 in shNA format.

In one embodiment of the nucleic acid molecules of the present invention, the nucleotide sequences are mammalian sequences that suppress the expression of amino acid sequences encoding for pro-inflammatory cytokine receptors. In another embodiment of the nucleic acid molecules of the present invention, the nucleotide sequences are mammalian sequences that suppress the expression of amino acid sequences encoding for type I IL-1β receptors. In another embodiment of the nucleic acid molecules of the present invention, the nucleotide sequence is a mammalian sequence that comprises SEQ ID NO: 23. In another embodiment of the nucleic acid molecules of the present invention, the nucleotide sequence is a mammalian sequence that comprises SEQ ID NO: 24. In another embodiment of the nucleic acid molecules of the present invention, the nucleotide sequence is a mammalian sequence that comprises SEQ ID NO: 25. In another embodiment of the nucleic acid molecules of the present invention, the nucleotide sequence is a mammalian sequence that comprises SEQ ID NO: 26. In another embodiment of the nucleic acid molecules of the present invention, the nucleotide sequence is a mammalian sequence that comprises SEQ ID NO: 27. In another embodiment of the nucleic acid molecules of the present invention, the nucleotide sequence is a mammalian sequence that comprises SEQ ID NO: 28. In another embodiment of the nucleic acid molecules of the present invention, the nucleotide sequence is a mammalian sequence that comprises SEQ ID NO: 23 in shNA format. In another embodiment of the nucleic acid molecules of the present invention, the nucleotide sequence is a mammalian sequence that comprises SEQ ID NO: 24 in shNA format. In another embodiment of the nucleic acid molecules of the present invention, the nucleotide sequence is a mammalian sequence that comprises SEQ ID NO: 25 in shNA format. In another embodiment of the nucleic acid molecules of the present invention, the nucleotide sequence is a mammalian sequence that comprises SEQ ID NO: 26 in shNA format. In another embodiment of the nucleic acid molecules of the present invention, the nucleotide sequence is a mammalian sequence that comprises SEQ ID NO: 27 in shNA format. In another embodiment of the nucleic acid molecules of the present invention, the nucleotide sequence is a mammalian sequence that comprises SEQ ID NO: 28 in shNA format.

In one embodiment of the nucleic acid molecules of the present invention, the nucleotide sequences are mammalian sequences that suppress the expression of amino acid sequences encoding for type II IL-1β receptors. In another embodiment of the nucleic acid molecules of the present invention, the nucleotide sequence is a mammalian sequence that comprises SEQ ID NO: 29. In another embodiment of the nucleic acid molecules of the present invention, the nucleotide sequence is a mammalian sequence that comprises SEQ ID NO: 30. In another embodiment of the nucleic acid molecules of the present invention, the nucleotide sequence is a mammalian sequence that comprises SEQ ID NO: 31. In another embodiment of the nucleic acid molecules of the present invention, the nucleotide sequence is a mammalian sequence that comprises SEQ ID NO: 32. In another embodiment of the nucleic acid molecules of the present invention, the nucleotide sequence is a mammalian sequence that comprises SEQ ID NO: 33. In another embodiment of the nucleic acid molecules of the present invention, the nucleotide sequence is a mammalian sequence that comprises SEQ ID NO: 34. In another embodiment of the nucleic acid molecules of the present invention, the nucleotide sequence is a mammalian sequence that comprises SEQ ID NO: 29 in shNA format. In another embodiment of the nucleic acid molecules of the present invention, the nucleotide sequence is a mammalian sequence that comprises SEQ ID NO: 30 in shNA format. In another embodiment of the nucleic acid molecules of the present invention, the nucleotide sequence is a mammalian sequence that comprises SEQ ID NO: 31 in shNA format. In another embodiment of the nucleic acid molecules of the present invention, the nucleotide sequence is a mammalian sequence that comprises SEQ ID NO: 32 in shNA format. In another embodiment of the nucleic acid molecules of the present invention, the nucleotide sequence is a mammalian sequence that comprises SEQ ID NO: 33 in shNA format. In another embodiment of the nucleic acid molecules of the present invention, the nucleotide sequence is a mammalian sequence that comprises SEQ ID NO: 34 in shNA format.

In one embodiment of the nucleic acid molecules of the present invention, the nucleotide sequences are mammalian sequences that suppress the expression of amino acid sequences encoding for type I TNFα receptors. In another embodiment of the nucleic acid molecules of the present invention, the nucleotide sequence is a mammalian sequence that comprises SEQ ID NO: 35. In another embodiment of the nucleic acid molecules of the present invention, the nucleotide sequence is a mammalian sequence that comprises SEQ ID NO: 36. In another embodiment of the nucleic acid molecules of the present invention, the nucleotide sequence is a mammalian sequence that comprises SEQ ID NO: 37. In another embodiment of the nucleic acid molecules of the present invention, the nucleotide sequence is a mammalian sequence that comprises SEQ ID NO: 38. In another embodiment of the nucleic acid molecules of the present invention, the nucleotide sequence is a mammalian sequence that comprises SEQ ID NO: 39. In another embodiment of the nucleic acid molecules of the present invention, the nucleotide sequence is a mammalian sequence that comprises SEQ ID NO: 40. In another embodiment of the nucleic acid molecules of the present invention, the nucleotide sequence is a mammalian sequence that comprises SEQ ID NO: 35 in shNA format. In another embodiment of the nucleic acid molecules of the present invention, the nucleotide sequence is a mammalian sequence that comprises SEQ ID NO: 36 in shNA format. In another embodiment of the nucleic acid molecules of the present invention, the nucleotide sequence is a mammalian sequence that comprises SEQ ID NO: 37 in shNA format. In another embodiment of the nucleic acid molecules of the present invention, the nucleotide sequence is a mammalian sequence that comprises SEQ ID NO: 38 in shNA format. In another embodiment of the nucleic acid molecules of the present invention, the nucleotide sequence is a mammalian sequence that comprises SEQ ID NO: 39 in shNA format. In another embodiment of the nucleic acid molecules of the present invention, the nucleotide sequence is a mammalian sequence that comprises SEQ ID NO: 40 in shNA format.

In one embodiment of the nucleic acid molecules of the present invention, the nucleotide sequences are mammalian sequences that suppress the expression of amino acid sequences encoding for type II TNFα receptors. In another embodiment of the nucleic acid molecules of the present invention, the nucleotide sequence is a mammalian sequence that comprises SEQ ID NO: 41. In another embodiment of the nucleic acid molecules of the present invention, the nucleotide sequence is a mammalian sequence that comprises SEQ ID NO: 42. In another embodiment of the nucleic acid molecules of the present invention, the nucleotide sequence is a mammalian sequence that comprises SEQ ID NO: 43. In another embodiment of the nucleic acid molecules of the present invention, the nucleotide sequence is a mammalian sequence that comprises SEQ ID NO: 44. In another embodiment of the nucleic acid molecules of the present invention, the nucleotide sequence is a mammalian sequence that comprises SEQ ID NO: 45. In another embodiment of the nucleic acid molecules of the present invention, the nucleotide sequence is a mammalian sequence that comprises SEQ ID NO: 46. In another embodiment of the nucleic acid molecules of the present invention, the nucleotide sequence is a mammalian sequence that comprises SEQ ID NO: 41 in shNA format. In another embodiment of the nucleic acid molecules of the present invention, the nucleotide sequence is a mammalian sequence that comprises SEQ ID NO: 42 in shNA format. In another embodiment of the nucleic acid molecules of the present invention, the nucleotide sequence is a mammalian sequence that comprises SEQ ID NO: 43 in shNA format. In another embodiment of the nucleic acid molecules of the present invention, the nucleotide sequence is a mammalian sequence that comprises SEQ ID NO: 44 in shNA format. In another embodiment of the nucleic acid molecules of the present invention, the nucleotide sequence is a mammalian sequence that comprises SEQ ID NO: 45 in shNA format. In another embodiment of the nucleic acid molecules of the present invention, the nucleotide sequence is a mammalian sequence that comprises SEQ ID NO: 46 in shNA format.

In one embodiment of the uses of the present invention, the nucleic acid molecules comprise the nucleotide sequence of SEQ ID NO: 4. In another embodiment of the uses of the present invention, the nucleic acid molecules comprise shNA that comprises the nucleotide sequence of SEQ ID NO: 47. In another embodiment of the uses of the present invention, the nucleic acid molecules comprise the nucleotide sequence of SEQ ID NO: 13. In another embodiment of the uses of the present invention, the nucleic acid molecules comprise shNA that comprises the nucleotide sequence of SEQ ID NO: 48.

The present invention also includes nucleic acid molecules that comprise the nucleotide sequence of SEQ ID NO: 4. In another embodiment, the nucleic acid molecules comprise a shNA sequence that comprises the nucleotide sequence of SEQ ID NO: 47. In another embodiment, the nucleic acid molecules comprise a sequence that comprises the nucleotide sequence of SEQ ID NO: 13. In another embodiment, the nucleic acid molecules comprise a shNA sequence that comprises the nucleotide sequence of SEQ ID NO: 48.

In another embodiment of the nucleic acid molecules of the present invention the molecule is administered locally to the perispinal region of the lumbar region of a spinal cord or is administered locally to the intrathecal space of the lumbar region of a spinal cord.

in another embodiment of the nucleic acid molecules of the present invention the molecule is administered locally from one of the administration routes selected from a catheter and drug pump, one or more local injections and polymer release.

The present invention also includes a system for treating pain that includes locally administering nucleic acid molecules that locally suppress the expression of amino acid sequences that yield pro-inflammatory cytokines and/or their receptors in the perispinal region or intrathecal space of the lumbar region of the spinal cord. The present invention also includes methods of using the systems of the present invention to treat acute or neuropathic pain associated with sciatica.

The present invention also includes systems. In one embodiment of the systems of the present invention the system comprises nucleic acid molecules comprising one or more sequences that suppress the expression of amino acid sequences encoding for inflammatory response proteins wherein the system is used to treat pain locally.

In another embodiment of the systems of the present invention, the inflammatory response proteins are pro-inflammatory cytokines selected from the group consisting of interleukin-1 beta ("IL-1β") and tumor necrosis factor alpha ("TNFα") or are pro-inflammatory cytokine receptors selected from the group consisting of type I IL-1β receptors, type II IL-1β receptors, type I TNFα receptors and type II TNFα receptors.

In another embodiment of the systems of the present invention, the pro-inflammatory cytokine is IL-1β and the nucleic acid molecules comprise mammalian nucleotide sequences selected from the group consisting of SEQ ID NO: 14; SEQ ID NO: 15; SEQ ID NO: 16; SEQ ID NO: 17; SEQ ID NO: 14 in shNA format; SEQ ID NO: 15 in shNA format; SEQ ID NO: 16 in shNA format; SEQ ID NO: 17 in shNA format and combinations thereof or the pro-inflammatory cytokine is TNFα and the nucleic acid molecules are mammalian nucleotide sequences selected from the group consisting of SEQ ID NO: 18; SEQ ID NO: 19; SEQ ID NO: 20; SEQ ID NO: 21; SEQ ID NO: 18 in shNA format; SEQ ID NO: 19 in shNA format; SEQ ID NO: 20 in shNA format; SEQ ID NO: 21 in shNA format and combinations thereof.

In another embodiment of the systems of the present invention, the pro-inflammatory cytokine receptor is a type I IL-1β receptor and the nucleic acid molecules are mammalian nucleotide sequences selected from the group consisting of SEQ ID NO: 23; SEQ ID NO: 24; SEQ ID NO: 25; SEQ ID NO: 26; SEQ ID NO: 27; SEQ ID NO: 28; SEQ ID NO: 23 in shNA format; SEQ ID NO: 24 in shNA format; SEQ ID NO: 25 in shNA format; SEQ ID NO: 26 in shNA format; SEQ ID NO: 27 in shNA format; SEQ ID NO: 28 in shNA format and combinations thereof.

In another embodiment of the systems of the present invention, the pro-inflammatory cytokine receptor is a type II IL-1β receptor and the nucleic acid molecules are mammallan nucleotide sequences selected from the group consisting of SEQ ID NO: 29; SEQ ID NO: 30; SEQ ID NO: 31; SEQ ID NO: 32; SEQ ID NO: 33; SEQ ID NO: 34; SEQ ID NO: 29 In shNA format: SEQ ID NO: 30 in shNA format; SEQ ID NO: 31 in shNA format; SEQ ID NO: 32 in shNA format; SEQ ID NO: 33 In shNA format; SEQ ID NO: 34 in shNA format and combinations thereof.

In another embodiment of the systems of the present invention, the pro-inflammatory cytokine receptor is a type I TNFα receptor and the nucleic acid molecules are mammallan nucleotide sequences selected from the group consisting of SEQ ID NO: 35; SEQ ID NO: 36; SEQ ID NO: 37; SEQ ID NO: 38; SEQ ID NO: 39; SEQ ID NO: 40; SEQ ID NO: 35 in shNA format; SEQ ID NO: 36 in shNA format; SEQ ID NO: 37 In shNA format; SEQ ID NO: 38 In shNA format; SEQ ID NO: 39 In shNA format; SEQ ID NO: 40 in shNA format and combinations thereof.

In another embodiment of the systems of the present invention, the pro-Inflammatory cytokine receptor is a type II TNFα receptor and the nucleic acid molecules are mammallan nucleotide sequences selected from the group consisting of SEQ ID NO: 41; SEQ ID NO: 42; SEQ ID NO: 43; SEQ ID NO: 44; SEQ ID NO: 45; SEQ ID NO: 46; SEQ ID NO: 41 in shNA format; SEQ ID NO: 42 in shNA format; SEQ ID NO: 43 in shNA format; SEQ ID NO: 44 in shNA format; SEQ ID NO: 45 in shNA format; SEQ ID NO: 46 in shNA format and combinations thereof.

In another embodiment of the systems of the present invention, the pain is acute or neuropathic. In another embodiment of the systems of the present invention, the pain Is sclatica.

In another embodiment of the systems of the present invention, the system comprises an administration form to administer the nucleic add molecules locally to the perispinal region of the lumbar region of a spinal cord or locally to the intrathecal space of the lumbar region of a spinal cord. In another embodiment of the systems of the present invention, the administration form is selected from one or more of the group consisting of a catheter and drug pump, one or more local injections and polymer release.

The present invention also includes kits as defined in the accompanying claims in one embodiment of the kits of the present invention the kit comprises nucleic acid molecules comprising one or more sequences that suppress the expression of amino acid sequences encoding for inflammatory response proteins and an administration form used to locally delivers the nucleic molecules.

In another embodiment of the kits of the present invention, the inflammatory response proteins are pro-inflammatory cytokines selected from the group consisting of interleukin-1 beta ("IL-1β") and tumor necrosis factor alpha ("TNFα") or are pro-inflammatory cytokine receptors selected from the group consisting of type I IL-1β receptors, type II IL-1β receptors, type I TNFα receptors and type II TNFα receptors.

In another embodiment of the kits of the present invention, the pro-inflammatory cytokine is IL-1β and the nucleic acid molecules comprise mammalian nucleotide sequences selected from the group consisting of SEQ ID NO: 14; SEQ ID NO: 15; SEQ ID NO: 16; SEQ ID NO: 17; SEQ ID NO: 14 in shNA format; SEQ ID NO: 15 in shNA format; SEQ ID NO: 16 in shNA format; SEQ ID NO: 17 in shNA format and combinations thereof or the pro-inflammatory cytokine is TNFα and the nucleic acid molecules are mammalian nucleotide sequences selected from the group consisting of SEQ ID NO: 18; SEQ ID NO: 19; SEQ ID NO: 20; SEQ ID NO: 21; SEQ ID NO: 18 in shNA format; SEQ ID NO: 19 in shNA format; SEQ ID NO: 20 in shNA format; SEQ ID NO: 21 in shNA format and combinations thereof.

In another embodiment of the kits of the present invention, the pro-inflammatory cytokine receptor is a type I IL-1β receptor and the nucleic acid molecules are mammalian nucleotide sequences selected from the group consisting of SEQ ID NO: 23; SEQ ID NO: 24; SEQ ID NO: 25; SEQ ID NO: 26; SEQ ID NO: 27; SEQ ID NO: 28; SEQ ID NO: 23 in shNA format; SEQ ID NO: 24 in shNA format; SEQ ID NO: 25 in shNA format; SEQ ID NO: 26 in shNA format; SEQ ID NO: 27 in shNA format; SEQ ID NO: 28 in shNA format and combinations thereof.

In another embodiment of the kits of the present invention, the pro-inflammatory cytokine receptor is a type II IL-1β receptor and the nucleic acid molecules are mammalian nucleotide sequences selected from the group consisting of SEQ ID NO: 29; SEQ ID NO: 30; SEQ ID NO: 31; SEQ ID NO: 32; SEQ ID NO: 33; SEQ ID NO: 34; SEQ ID NO: 29 in shNA format; SEQ ID NO: 30 in shNA format; SEQ ID NO: 31 in shNA format; SEQ ID NO: 32 in shNA format; SEQ ID NO: 33 in shNA format; SEQ ID NO: 34 in shNA format and combinations thereof.

In another embodiment of the kits of the present invention, the pro-inflammatory cytokine receptor is a type I TNFα receptor and the nucleic acid molecules are mammalian nucleotide sequences selected from the group consisting of SEQ ID NO: 35; SEQ ID NO: 36; SEQ ID NO: 37; SEQ ID NO: 38; SEQ ID NO: 39; SEQ ID NO: 40; SEQ ID NO: 35 in shNA format; SEQ ID NO: 36 in shNA format; SEQ ID NO: 37 in shNA format; SEQ ID NO: 38 in shNA format; SEQ ID NO: 39 in shNA format; SEQ ID NO: 40 in shNA format and combinations thereof.

In another embodiment of the kits of the present invention, the pro-inflammatory cytokine receptor is a type II TNFα receptor and the nucleic acid molecules are mammalian nucleotide sequences selected from the group consisting of SEQ ID NO: 41; SEQ ID NO: 42; SEQ ID NO: 43; SEQ ID NO: 44; SEQ ID NO: 45; SEQ ID NO: 46; SEQ ID NO: 41 in shNA format; SEQ ID NO: 42 in shNA format; SEQ ID NO: 43 in shNA format; SEQ ID NO: 44 in shNA format; SEQ ID NO: 45 in shNA format; SEQ ID NO: 46 in shNA format and combinations thereof.

In another embodiment of the kits of the present invention, the pain is acute or neuropathic. In another embodiment of the kits of the present invention, the pain is sciatica.

In another embodiment of the kits of the present invention, the administration form is used to administer the nucleic acid molecules locally to the perispinal region of the lumbar region of a spinal cord or locally to the intrathecal space of the lumbar region of a spinal cord. In another embodiment of the kits of the present invention, the administration form is selected from one or more of the group consisting of a catheter and drug pump, one or more local injections and polymer release.

### BRIEF DESCRIPTION OF THE FIGURES

FIG. **1** shows behavioral pain sensitivity data following the intraperitoneal or subcutaneous administration of protein-based pro-inflammatory cytokine inhibitors.

FIG. **2** shows behavioral pain sensitivity data following intraperitoneal or local administration of protein-based pro-inflammatory cytokine inhibitors.

FIG. **3** shows behavioral pain sensitivity data following polymer-mediated local administration of protein-based proinflammatory cytokine inhibitors.

FIG. **4** shows a target plasmid for nucleic acid sequence characterization in HEK293 cells.

FIG .**5** shows rat IL-1β mRNA suppression after nucleic acid sequence transfection studies.

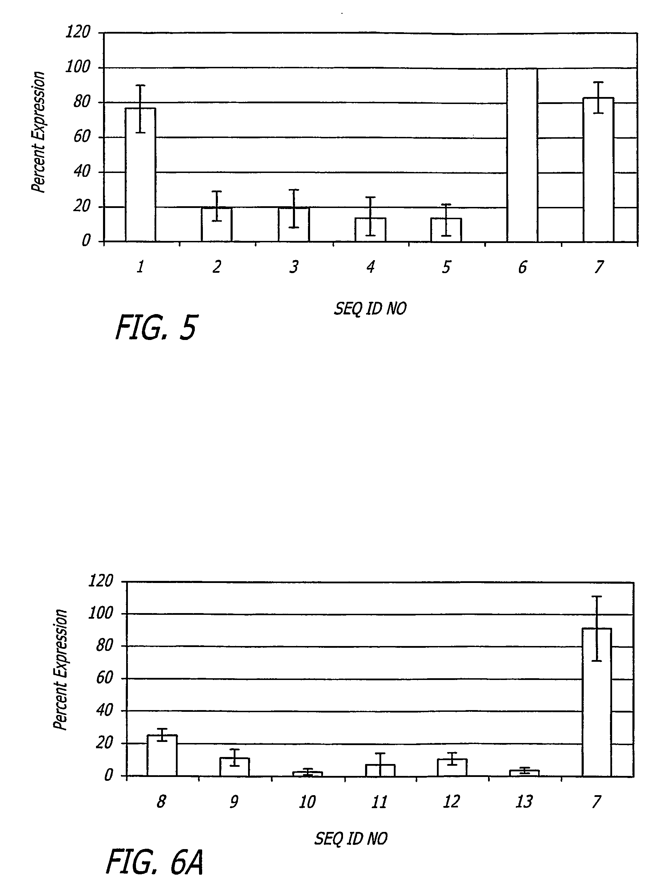

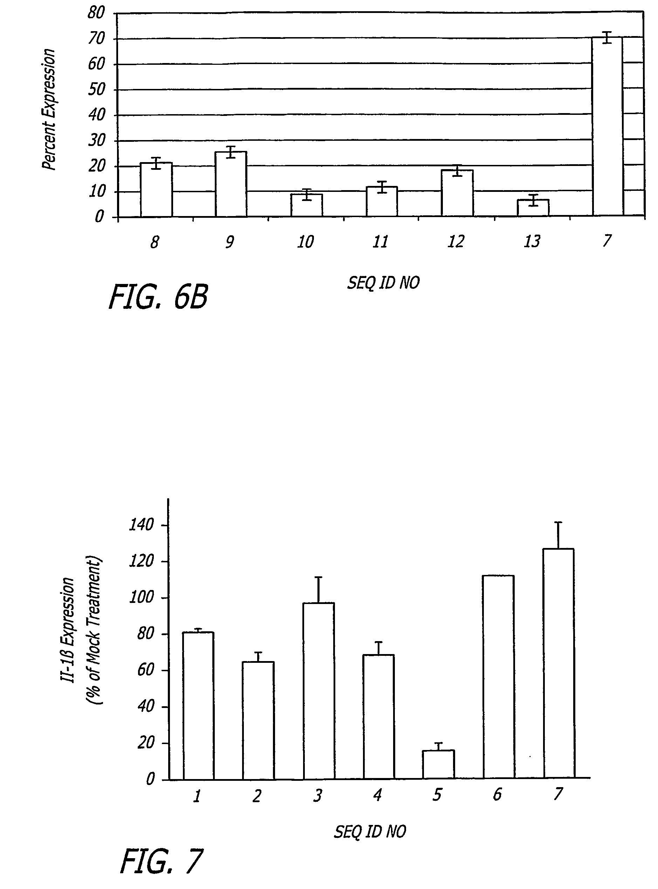

FIG .**6** shows rat TNFα mRNA (FIG. **6A**) and protein suppression (FIG. **6B**) after nucleic acid sequence transfection studies.

FIG. **7** shows rat IL-1β protein suppression after nucleic acid sequence transfection studies in NR8383 cells.

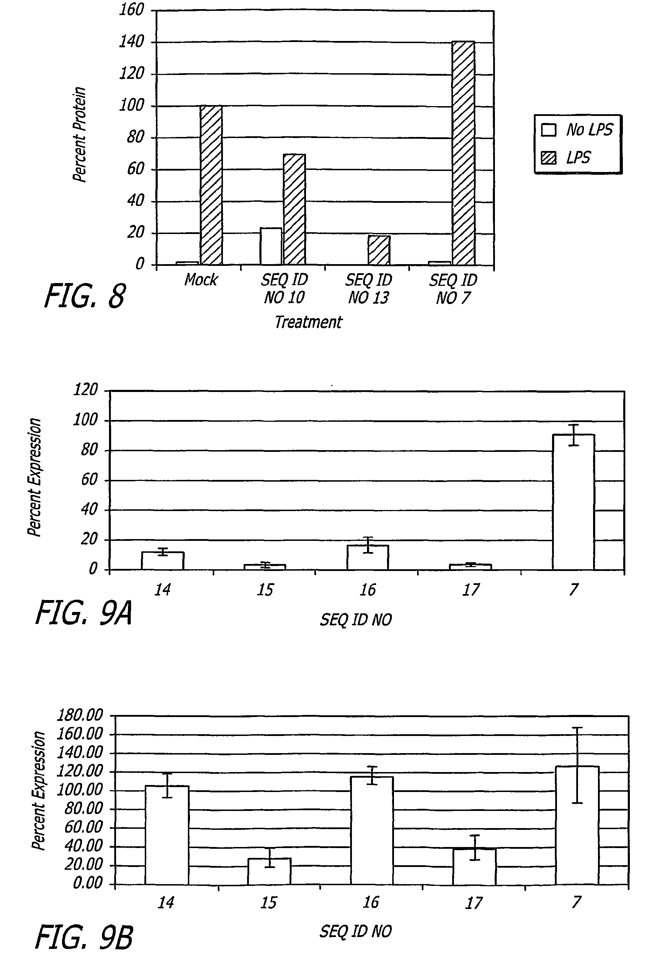

FIG. **8** shows endogenous rat TNFα protein suppression after nucleic acid sequence transfection studies in NR8383 cells.

FIG. **9** shows human IL-1β mRNA (FIG. **9A**) and protein suppression (FIG. **9B**) after nucleic acid sequence transfection studies.

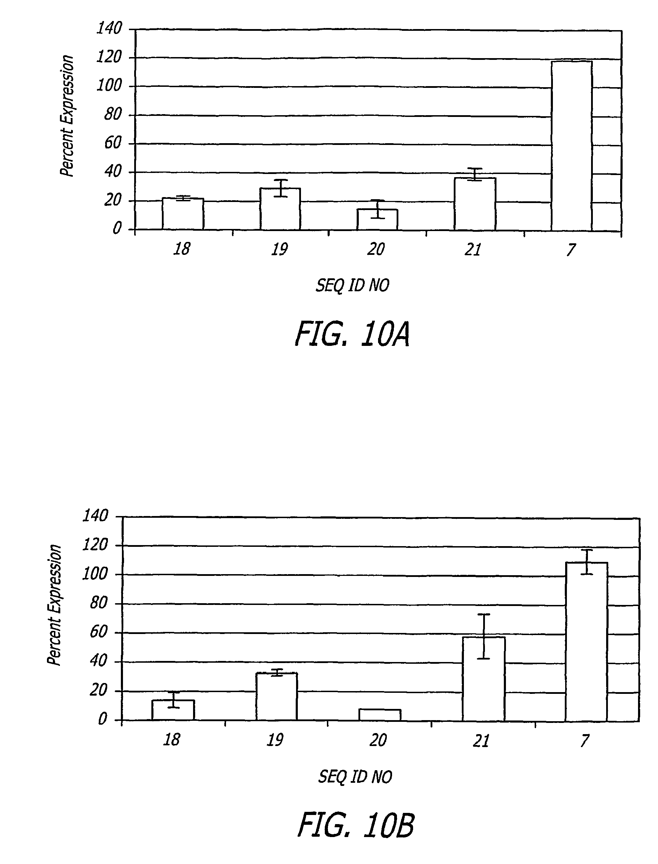

FIG. **10** shows human TNFα mRNA (FIG. **10A**) and protein suppression (FIG. **10B**) after nucleic acid sequence transfection studies.

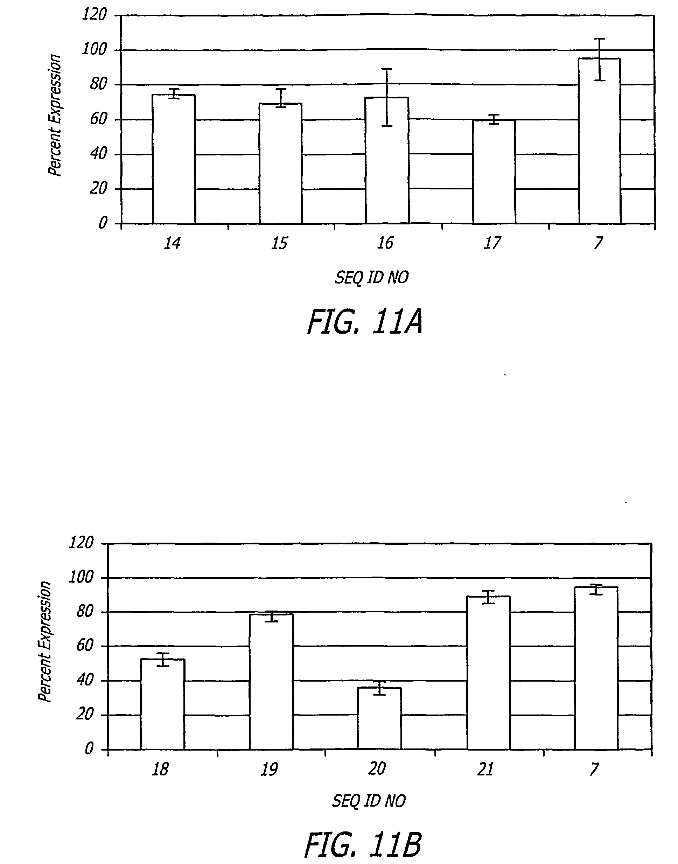

FIG. **11** shows endogenous human IL-1β. protein suppression (FIG.**11A**) and human TNFα protein suppression (FIG. **11B**) after nucleic acid sequence transfection studies in primary human monocytes.

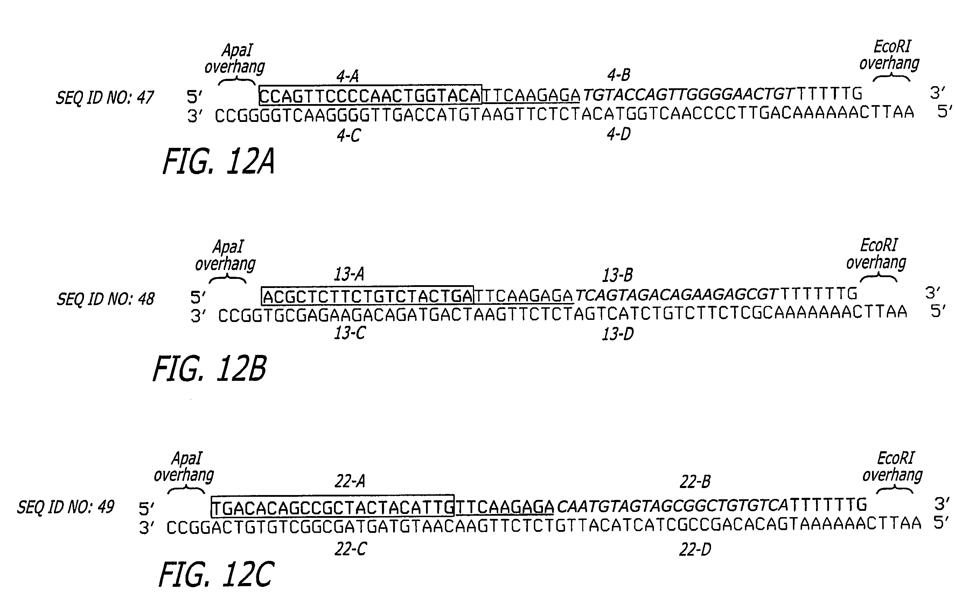

FIG. **12** depicts the structure and construction of anti-r/hIL-1β (FIG. **12A**); anti-rTNFα (FIG. **12B**) and control-2 (FIG. **12C**) shNA sequences.

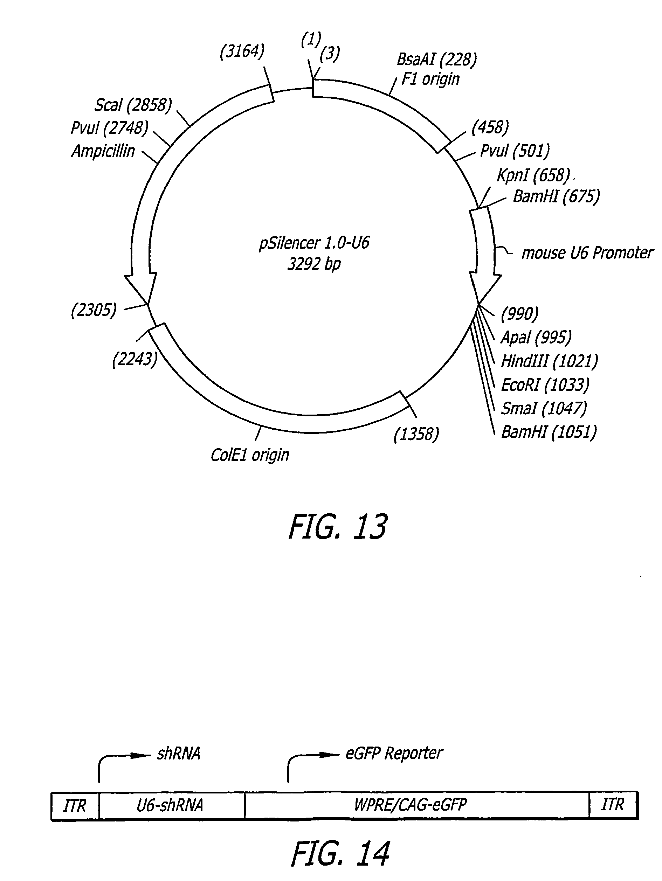

FIG. **13** shows a schematic description of the pSilencer 1.0-U6 plasmid used in the preparation of rat IL-1β, rat TNFα and control-2 shNA sequences.

FIG. **14** shows the format of AAV viral constructs.

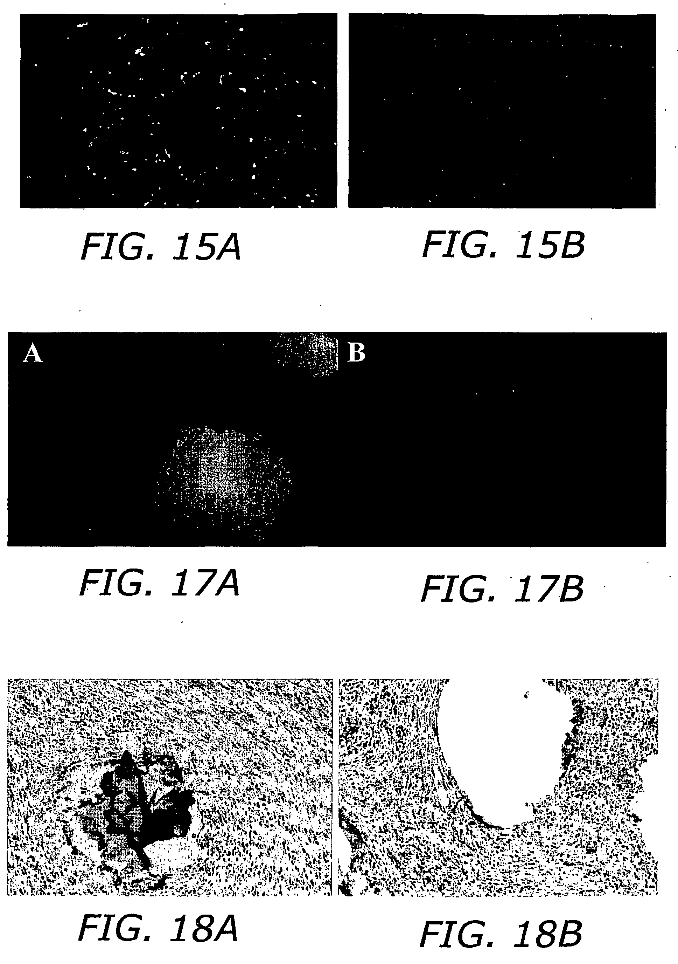

FIG. **15** shows GFP fluorescence results regarding *in vitro* cellular transduction by an AAV viral construct made in accordance with the disclosure of the present invention.

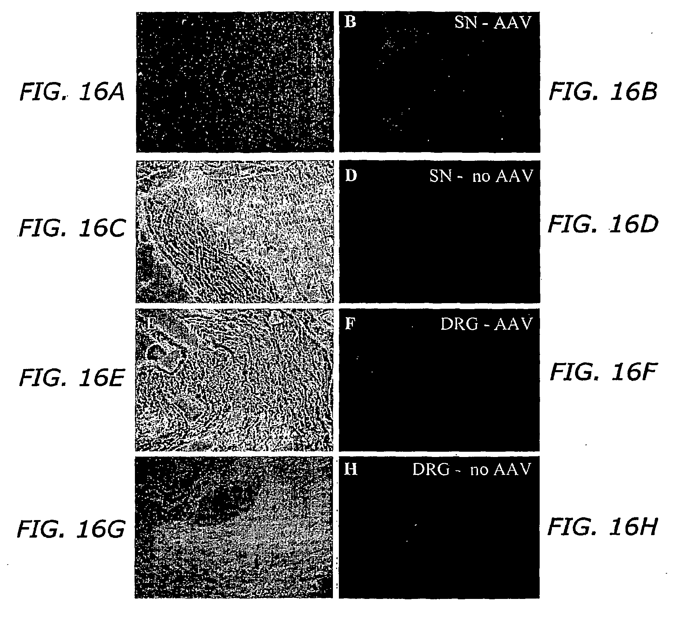

FIG. **16** shows brightfield and fluorescent image analysis results regarding *in vivo* cellular transduction by an AAV viral construct made in accordance with the disclosure of the present invention in animals with sciatic nerve cryo-injuries.

FIG. **17** shows GFP fluorescence results regarding *in vivo* cellular transduction by an AAV viral construct made in accordance with the disclosure of the present invention in chronic constriction injury ("CCI") animals.

FIG. **18** shows GFP immunohistochemical ("IHC") staining results regarding *in vivo* cellular transduction by an AAV viral construct made in accordance with the disclosure of the present invention in CCI animals.

### DEFINITION OF TERMS

The term "SEQ ID NO: X" (where X is any number from 1 to 46) refers to, in one embodiment, each number's sequence as defined in Table 2 (identified sequence). SEQ ID NO: X must also be read to encompass sequences that would hybridize with the complementary strand of a sequence set forth in SEQ ID NOS: 1-46 and reduce that SEQ ID NO:'s target gene expression by 20% or more in a cell type selected from, without limitation, rat monocytes, rat macrophages, human monocytes or human macrophages or in a cell line selected from, without limitation, NR8383 cells, HEK293 cells or HeLa cells. Under this definition, claimed sequences can include at least 99% sequence homology with the identified sequence; at least 98% sequence homology with the identified sequence; at least 95% sequence homology with the identified sequence; at least 90% sequence homology with the identified sequence; or at least 85% sequence homology with the identified sequence.

The terms "nucleic acid'" or "nucleic acid molecules" refer to deoxyribonucleotides or ribonucleotides and polymers thereof in either single- or double-stranded form, composed of monomers (nucleotides) containing a sugar, phosphate and a base that is either a purine or pyrimidine. Unless specifically limited, the term encompasses nucleic acids containing known analogs of natural nucleotides that have similar binding properties as the reference nucleic acid and are metabolized in a manner similar to naturally occurring nucleotides. Unless otherwise indicated, a particular nucleic acid sequence also encompasses conservatively modified variants thereof and complementary sequences, as well as the sequence explicitly indicated. The nucleic acid molecules of the present invention can include any type of nucleic acid molecule capable of mediating RNA interference, such as, without limitation, short interfering nucleic acid (siNA), short hairpin nucleic acid (shNA), short interfering RNA (siRNA), short hairpin RNA (shRNA), micro-RNA (miRNA), and double-stranded RNA (dsRNA). The nucleic acid molecules of the present invention also include similar DNA sequences. Further, the nucleic acid and nucleic acid molecules of the present invention can contain unmodified or modified nucleotides. Modified nucleotides refer to nucleotides which contain a modification in the chemical structure of a nucleotide base, sugar and/or phosphate. Such modifications can be made to improve the stability and/or efficacy of nucleic acid molecules and are described in patents and publications such as United States Patent Number ("USPN") 6,617,438, USPN 5,334,711; USPN 5,716,824; USPN 5,627,053; United States Patent Application Number 60/082,404, International Patent Cooperation Treaty Publication Number ("PCTPN") WO 98/13526; PCTPN WO 92/07065; PCTPN WO 03/070897; PCTPN WO 97/26270; PCTPN WO 93/15187; Beigelman et al., 1995, J. Biol. Chem., 270, 25702; Usman and Cedergren, 1992, TIBS. 17, 34; Usman et al., 1994, Nucleic Acids Symp. Ser. 31, 163; Burgin et al., 1996, Biochemistry, 35, 14090; Perrault et al. Nature, 1990, 344, 565-568; Pieken et al. Science, 1991, 253, 314-317; Usman and Cedergren, Trends in Biochem. Sci., 1992, 17, 334-339; Karpeisky et al., 1998, Tetrahedron Lett., 39, 1131; Earnshaw and Gait, 1998, Biopolymers (Nucleic Acid Sciences), 48, 39-55; Verma and Eckstein, 1998, Annu. Rev. Biochem., 67, 99-134; Burlina et al., 1997, Bioorg. Med. Chem., 5, 1999-2010; Limbach et al., 1994, Nucleic Acids Res. 22, 2183; and Burgin et al., 1996, Biochemistry, 35, 14090. Such patents and publications describe general methods and strategies to modify nucleic acid molecules

The phrase "expression cassette" as used herein means a nucleic acid sequence capable of directing expression of a particular nucleotide sequence in an appropriate host cell, with additional sequences that facilitate appropriate transcription of the nucleic acid sequence of interest. In addition to the nucleotide sequence of interest, the expression cassette can include a promoter operably linked to the nucleotide sequence of interest that also can be operably linked to termination and/or polyadenylation signals. The expression cassette also can include expression enhancers. The expression cassette including the nucleotide sequence of interest can be chimeric. The expression cassette also can be one that is naturally occurring but has been obtained in a recombinant form useful for heterologous expression. The expression of the nucleotide sequence in the expression cassette can be under the control of a constitutive promoter or of a regulatable promoter that initiates transcription only when the host cell is exposed to some particular stimulus. In the case of a multicellular organism, the promoter also can be specific to a particular tissue or organ or stage of development.

The term "promoter" refers to a nucleotide sequence, usually upstream (5 prime) of the nucleotide sequence of interest, which directs and/or controls expression of the nucleotide sequence of interest by providing for recognition by RNA polymerase and other factors required for proper transcription. As used herein, the term "promoter" includes (but is not limited to) a minimal promoter that is a short DNA sequence comprised of a TATA-box and other sequences that serve to specify the site of transcription initiation, to which regulatory elements are added for control of expression. The term "promoter" also refers to a nucleotide sequence that includes a minimal promoter plus regulatory elements that is capable of controlling the expression of a coding sequence or functional RNA. This type of promoter sequence consists of proximal and more distal upstream elements, the latter elements often referred to as enhancers. The term "enhance" refers to a DNA sequence that can stimulate promoter activity and can be an innate element of the promoter or a heterologous element inserted to enhance the level or tissue specificity of a promoter. Enhancers are capable of operating in both orientations (normal or flipped), and are capable of functioning even when moved either upstream or downstream of the promoter. Both enhancers and other upstream promoter elements bind sequence-specific DNA-binding proteins that mediate their effects. Promoters can be derived in their entirety from a native gene, or be composed of different elements derived from different promoters found in nature, or even be comprised of synthetic DNA segments. A promoter also can contain DNA sequences that are involved in the binding of protein factors that control the effectiveness of transcription initiation in response to physiological or developmental conditions. Specific promoters used in accordance with the present invention can include, for example and without limitation pol II promoters (including, without limitation cytomegalovirus ("CMV") promoters, chicken β-actin ("CBA") promoters, Rous sarcoma virus ("RSV") promoters and neuron-specific enolase ("NSE") promoters). Furthermore, specific promoters used in accordance with the present invention can include, for example and without limitation, pol III promoters (including, without limitation, human H1 and human or murine U6 promoters, as well as H1 and U6 promoters engineered to be expressed in a regulated way such as described in United States Patent Application Publication Number 2005/0064489).

The term "vector" is defined to include any virus, as well as any plasmid, cosmid, phage, binary vector, or segment of nucleic acid (DNA or RNA) in double or single stranded linear or circular form that may or may not be self-transmissible or mobilizable, and that can transform eukaryotic host cells either by integration into the cellular genome or by existing extrachromosomally with or without the ability to replicate (e.g., autonomous replicating plasmid with an origin of replication).

### DETAILED DESCRIPTION OF THE INVENTION

In order to use RNA interference ("RNAi") to treat acute or neuropathic pain locally, it was first necessary to identify both behavioral pain models sensitive to pro-inflammatory cytokine and receptor suppression as well as nucleic acid sequences that effectively suppress the expression of interleukin-1 beta ("IL-1β"), tumor necrosis factor alpha ("TNFα") and their receptors. After identification of effective nucleic acid sequences, a subset of these sequences was reformatted into shNA sequences. A description of the behavioral pain models will be provided first, followed by a description of the methods used to identify nucleic acid sequences that effectively suppress the expression of IL-1β and TNFα. Third, a description of the shNA sequences will be provided along with evidence of their viral transduction *in vitro* and *in vivo.* Finally, a description of an siRNA-mediated reduction of a pain-associated behavior in an *in vivo* model of neuropathic pain will be presented.

*Behavioral Pain Model - Chronic Constriction Injury*

The behavioral animal model of chronic constriction injury ("CCI") was chosen to evaluate the efficacy of pro-inflammatory cytokine and receptor suppression as a pain treatment. This model may mimic pain associated with sciatica in humans. To induce CCI, each animal was anesthetized by intraperitoneal ("i.p.") injection of sodium pentobarbital at a dose of 60mg/kg body weight. The animal's right common sciatic nerve was exposed and freed from adherent tissue at mid-thigh by separating the muscle (biceps femoris) by blunt dissection. Four loose ligatures were placed 1 mm apart, using chromic gut (4-0 absorbable suture, Jorgensen Laboratories Inc., Loveland, CO).

Animals were randomly assigned to treatment groups and administered control or test compounds as indicated in the table below. Animals received either vehicle (Phosphate Buffered Saline; PBS), a positive control compound such as Gabapentin^{®} (Depomed, Inc., Menlo Park, CA), a protein based TNFα inhibitor such as Enbrel^{®} (Immunex Corp., Seattle, WA) or Remicade^{®} (Centocor, Inc., Malvern, PA), or an IL-1 receptor antagonist such as Kineret^{®} (Amgen, Inc., Thousand Oaks, CA), starting on Day 1 (day of CCI surgery) as shown in Table 1:

**Table 1.**

| Compound | Route of Admin | Frequency of administration | Low Dose | High Dose |
|---|---|---|---|---|
| Vehicle (PBS) | Intraperitoneal ("IP") injection | Every 3 days | - | n/a |
| Gabapentin^{®} | Subcutaneous ("SC") injection | 1 hour prior to each behavioral test | 90 mg/kg | n/a |
| Enbrel^{®} | IP injection | Every 3 days | 2.4 mg/kg | 8 mg/kg |
| Remicade^{®} | IP injection | Every 8 days | 2.4 mg/kg | 8 mg/kg |
| Kineret^{®} | IP injection | Every day | 1 mg/kg | 10 mg/kg |

Animal behavioral testing was conducted on Days 7, 14, and 21 after CCI. In the thermal hyperalgesia test, animals were placed in the clear plastic chamber of the plantar analgesia instrument and allowed to acclimate to the environment for 15 minutes. After the acclimation period, a radiant (heat) beam source stimulus was applied to the CCI hind paw of each animal. The heat source device was set at an intensity of 50, and a maximum latency period of 15 seconds was set to prevent tissue damage according to the recommendations of the instrument manufacturer. If a paw withdrawal occurred within the 15 second period, an automated control interrupted both the stimulus and timer, turning off the radiant beam and recording the latency of time to paw withdrawal. Data was analyzed using a one-way analysis of variance at each test day.

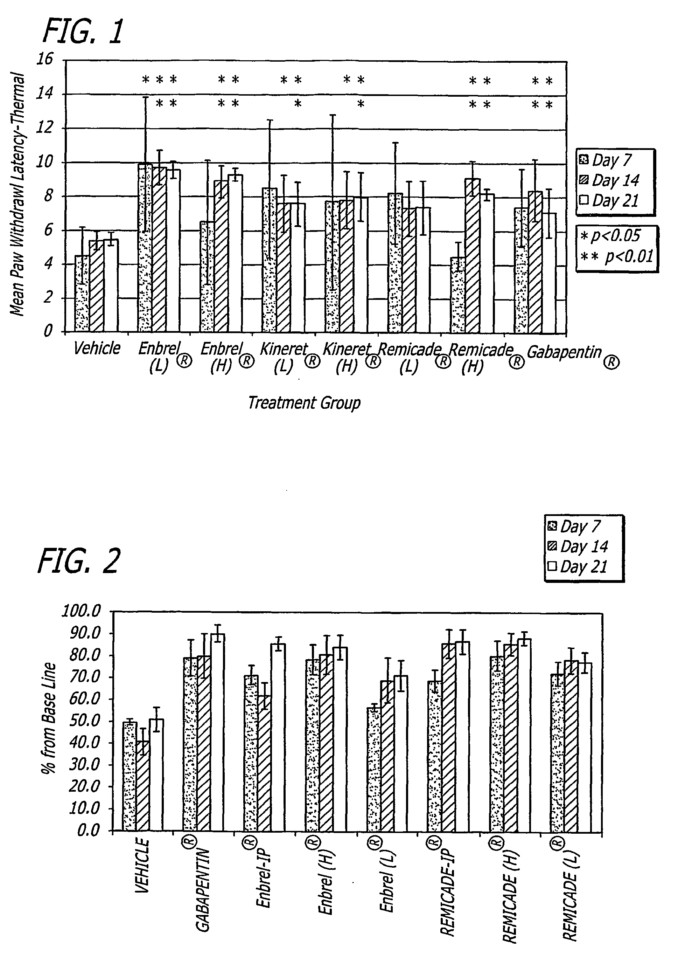

FIG. **1** demonstrates that the protein-based pro-inflammatory cytokine inhibitors were effective to inhibit pain associated with CCI. Animals that received vehicle showed a mean paw withdrawal latency of about five seconds. Animals that received a protein-based pro-inflammatory cytokine inhibitor showed mean paw withdrawal latencies of between about six to ten seconds. This data demonstrates that CCI combined with use of a plantar analgesia instrument provides a behavioral animal model that can demonstrate decreased sensitivity to pain (effective pain treatment) due to suppressing the actions of pro-inflammatory cytokines.

A second behavioral experiment was undertaken to determine if the local administration of protein-based pro-inflammatory cytokine inhibitors would inhibit pain as effectively as the previously-described systemic administration routes. In this experiment, the effectiveness of pro-inflammatory cytokine inhibitors administered through a subcutaneous Alzet^{®} osmotic pump (Model 2002, Durect Corp., Cupertino, CA) were compared with the i.p. administration of the same inhibitors. First, animals underwent CCI. Following CCI, animals were randomly assigned to the following treatment groups: locally-administered vehicle only (control); i.p. Gabapentin^{®} administered 1 hour prior to behavioral testing (positive control); i.p. Enbrel^{®} (2.4 mg/kg) administered on Days 1 (about 30 minutes after CCI injury), 4, 7, 10, 13, 16, and 19; locally-administered Enbrel^{®} (high dose: 10.0 µg/hr) on a continuous infusion with pump exchange on Day 10; locally-administered Enbrel^{®} (low dose: 1.0 µg/hr) on a continuous infusion with pump exchange on Day 10; i.p. Remicade^{®} (2.4 mg/kg) administered on Days 1 (about 30 minutes after CCI injury), 9 and 17; locally-administered Remicade^{®} (high dose: 3.75 µg/hr) on a continuous infusion with pump exchange on Day 10; and locally-administered Remicade^{®} (low dose: 0.375 µg/hr) on a continuous infusion with pump exchange on Day 10. Note that animals receiving compounds via i.p. injection also were implanted with a catheter/Alzet^{®} osmotic pump. These pumps were filled with vehicle solution only.

Paw withdrawal latencies were measured on days 7, 14 and 21 post-CCI surgeries. As shown in FIG.**2**, the data demonstrated that the positive control (i.p. Gabapentin^{®}) produced a significant increase in paw withdrawal latencies on all three test days. Animals receiving i.p. Enbrel^{®} or the high dose of locally-administered Enbrel^{®} showed significant increases in paw withdrawal latencies on all three test days. Animals receiving the low dose of locally-administered Enbrel^{®} showed significant increases in paw withdrawal latencies on Days 14 and 21 of testing. All animals receiving Remicade^{®} regardless of administration route or dose showed significant increases in paw withdrawal latencies on all three test days.

A third behavioral experiment was undertaken to determine if polymer-mediated local administration of a protein-based pro-inflammatory cytokine inhibitor would inhibit pain as effectively as systemic and/or pump-based local administration methods. In this experiment, the effectiveness of a pro-inflammatory cytokine inhibitor (Enbrel^{®}) administered by perineural implant of two different controlled release polymer formulations was compared with the local (via Alzet^{®} osmotic pump) administration of the same inhibitor. The polymer formulations included Poly(DL-lactide-co-glycolide) 50/50 (PLGA) rods and Polyorthoester (POE) microspheres loaded with Enbrel^{®}.

As described in other behavioral studies, animals underwent CCI. Following CCI, animals were randomly assigned to the following treatment groups: no treatment (injury only control); Gabapentin^{®} 60 mg/kg administered subcutaneously 1 hour prior to behavioral testing (positive control); locally-administered Enbrel^{®} (1.0 µg/hr) via pump with a continuous infusion and pump reservoir exchange on Day 10; locally-administered Enbrel^{®} PLGA polymer rods (Enbrel^{®} elution: 2-3 µg/hr) implanted perineurally next to the CCI ligation; locally-administered Enbrel^{®} POE microspheres (Enbrel^{®} elution: 2-3 µg/hr) implanted perineurally next to the CCI ligation; PLGA polymer rods containing no inhibitor (unloaded polymer rod control) implanted perineurally next to the CCI ligation; and POE microspheres containing no inhibitor (unloaded polymer microsphere control) implanted perineurally next to the CCI ligation.

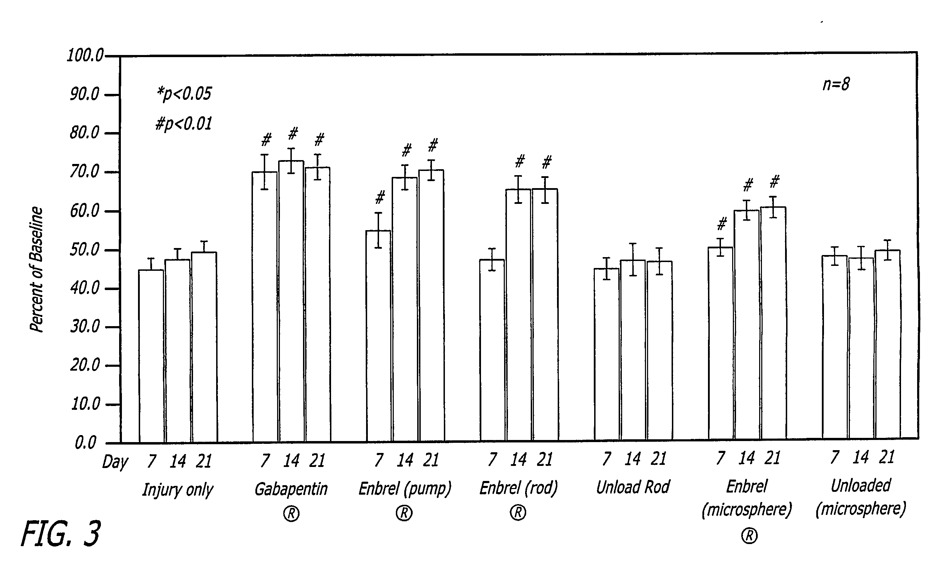

Paw withdrawal latencies following a thermal stimulus were measured on Days 7, 14 and 21 post-CCI surgeries. As shown in FIG. 3, the data demonstrated that the positive control (Gabapentin^{®}) produced a significant increase in paw withdrawal latencies on all three test days. Animals receiving locally-administered Enbrel^{®} via a pump showed significant increases in paw withdrawal latencies on Days 14 and 21 of testing. Animals receiving the Enbrel^{®} via either a PLGA polymer rod implant or a POE microsphere implant showed significant increases in paw withdrawal latencies on Days 14 and 21 of testing. The Enbrel^{®} POE microsphere-treated animals also showed significant increases in withdrawal latency on Day 7 of testing. Animals receiving unloaded PLGA rods or POE microspheres had no changes in their paw withdrawal latencies relative to the untreated (injury only) animals on any day of testing, thus demonstrating the safety of the polymer implants for delivery of anti-inflammatory mediators locally to a site of peripheral nerve injury.

The data from these behavioral studies further indicates that protein-based inhibitors of pro-inflammatory cytokines are effective at treating pain after CCI. Moreover, the local administration of these inhibitors via pump or implanted controlled release polymer is as effective as their systemic administration. This data suggests that the local inhibition of pro-inflammatory cytokines treats pain associated with damage to the sciatic nerve.

*Identification of Nucleic Acid Sequences that Suppress Production of IL-1β, TNFα and Their Receptors*

In addition to identifying an appropriate behavioral model to test the effectiveness of inhibiting pro-inflammatory cytokines and their receptors to treat pain, it also was necessary to identify nucleic acid sequences that effectively suppress the expression of IL-1β, TNFα and their receptors.

Initially, potential synthetic nucleic acid suppressor sequences for IL-1β, TNFα and their receptors were identified using online software (http://www.dharmacon.com/) and also obtained from Dharmacon^{®} (Dharmacon Research, Inc., Boulder, CO). These nucleic acid sequences are identified in Table 2:

**Table 2.**

| **SEQ ID NO:** | **Nucleic Acid Sequence Name** | **Target Gene*** | **Nucleotide Base Sequence** | **Position in cDNA** |
|---|---|---|---|---|
| 1 | L1 | rIL-1β | GAATGACCTGTTCTTTGAG | 130-148 |
| 2 | L2 | rIL-1β | TGAAAGCTCTCCACCTCAA | 501-519 |
| 3 | L3 | rIL-1β | GGTACATCAGCACCTCTCA | 783-801 |
| 4 | L4 | rIL-1β | CAGTTCCCCAACTGGTACA | 770-788 |
| 5 | L5 | rIL-1β | AGATAGAAGTCAAGACCAA | 732-750 |
| 6 | L6 | rIL-1β | TAAGCCAACAAGTGGTATT | 531-549 |
| 7 | CTRL | Off Target | AAGACTGTGGCTACAACATTC | n/a |
| 8 | RP1 | rTNFα | AAGCATGATCCGAGATGTG | 12-30 |
| 9 | RP2 | rTNFα | CATGATCCGAGATGTGGAA | 15-33 |
| 10 | RP3 | rTNFα | ACCACGCTCTTCTGTCTAC | 133-151 |
| 11 | RP4 | rTNFα | CCACGCTCTTCTGTCTACT | 134-152 |
| 12 | RP5 | rTNFα | CACGCTCTTCTGTCTACTG | 135-153 |
| 13 | RP6 | rTNFα | ACGCTCTTCTGTCTACTGA | 136-154 |
| 14 | HLA | hIL-1β | GTGAAATGATGGCTTATTA | 113-131 |
| 15 | HLB | hIL-1β | CGATGCACCTGTACGATCA | 432-450 |
| 16 | HLC | hIL-1β | TAACTGACTTCACCATGCA | 863-881 |
| 17 | HLD | hIL-1β | GAACCTATCTTCTTCGACA | 388-406 |
| 18 | HTAA | hTNFα | CCAGGGACCTCTCTCTAAT | 360-378 |
| 19 | HTBB | hTNFα | AGGGACCTCTCTCTAATCA | 362-380 |
| 20 | HTCC | hTNFα | GCCTGTAGCCCATGTTGTA | 430-448 |
| 21 | HTDD | hTNFα | TGTAGCCCATGTTGTAGCA | 433-451 |
| 22 | CTRL-2 | Off Target | TGACACAGCCGCTACTACATTG | n/a |
| 23 | IRI-1 | hIL-1β RI | ACATTACTATTGCGTGGTA | 358-376 |
| 24 | IRI-2 | hIL-1β RI | GAATGAGCCTAACTTATGT | 427-445 |
| 25 | IRI-3 | hIL-1β RI | ATAGGCTCATCGTGATGAA | 618-636 |
| 26 | IRI-4 | hIL-1β RI | GAACTATACTTGTCATGCA | 658-676 |
| 27 | IRI-5 | hIL-1β RI | CGACGGTCACCTTCATCTA | 1706-1724 |
| 28 | IRI-6 | hIL-1β RI | ACGGTCACCTTCATCTAAA | 1708-1726 |
| 29 | IRII-1 | hIL-1β RII | ACCTACGTCTGCACTACTA | 542-560 |
| 30 | IRII-2 | hIL-1β RII | TCCTGCCGTTCATCTCATA | 624-642 |
| 31 | IRII-3 | hIL-1β RII | CCACTCACTTACTCGTACA | 795-813 |
| 32 | IRII-4 | hIL-1β RII | ACATTGAAGTGCCATTGAT | 1143-1161 |
| 33 | IRII-5 | hIL-1β RII | ACGTCTGCACTACTAGAAA | 546-564 |
| 34 | IRII-6 | hIL-1β RII | CTACTAGAAATGCTTCTTA | 555-573 |
| 35 | TRI-1 | hTNFα RI | CGGCATTATTGGAGTGAAA | 678-696 |
| 36 | TRI-2 | hTNFα RI | ACAAAGGAACCTACTTGTA | 469-487 |
| 37 | TRI-3 | hTNFα RI | AACCAGTACCGGCATTATT | 669-687 |
| 38 | TRI-4 | hTNFα RI | AATTCGATTTGCTGTACCA | 444-462 |
| 39 | TRI-5 | hTNFα RI | TTTAATGTATCGCTACCAA | 974-992 |
| 40 | TRI-6 | hTNFα RI | GAGCTTGAAGGAACTACTA | 1053-1071 |
| 41 | TRII-1 | hTNFα RII | ACATGCCGGCTCAGAGAAT | 204-222 |
| 42 | TRII-2 | hTNFα RII | GCCGGCTCAGAGAATACTA | 208-226 |
| 43 | TRII-3 | hTNFα RII | CATCAGACGTGGTGTGCAA | 556-574 |
| 44 | TRII-4 | hTNFα RII | CTCACTTGCCTGCCGATAA | 991-1009 |
| 45 | TRII-5 | hTNFα RII | TCACTTGCCTGCCGATAAG | 992-1010 |
| 46 | TRII-6 | hTNFα RII | ACACGACTTCATCCACGGA | 601-619 |

| | | | | |
|---|---|---|---|---|
| r=rat; h=human | | | | |

Note that, as will be understood by one of skill in the art, the nucleic acid molecules of the present invention include the sequences in the perceding table, the reverse complement of these sequences and RNA-based sequences including uracils in the place of the listed thymidines. Thus, the sequences in the preceding table may be considered target sequences as well as sequences included in the nucleic acid molecules of the present invention.

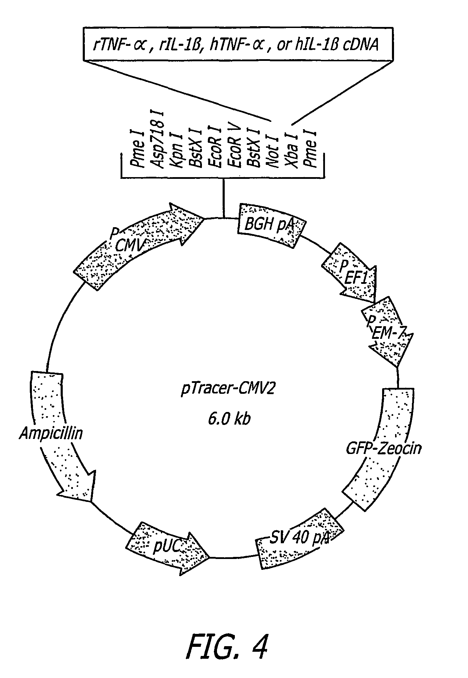

To identify which of the above identified nucleic acid sequences most effectively suppressed rat and human IL-1β or TNFα expression, an *in vitro* co-transfection assay system was developed. Referring to FIG. **4**, rat IL-1β, human IL-1β, rat TNFα, or human TNFα gene sequences were subcloned into pTracer^{™}-CMV2 (Invitrogen, Corp., Carlsbad, CA) to generate pTracer- rIL-1β, pTracer- hIL-1β, pTracer-rTNFα, and pTracer-hTNFα. The recombinant plasmid also included a GFP-Zeocin reporter gene for transfection efficiency normalization. The CMV promoter directed constitutive expression of the target genes (rat and human IL-1β or TNFα) while the EF1 promoter directed constitutive expression of the GFP-Zeocin reporter gene.

The generated recombinant plasmids were used to facilitate screening of nucleic acid sequences by co-transfection into a eukaryotic cell line. Specifically, HEK293 or HeLa cell cultures at 60-70% confluency were co-transfected with the appropriate target plasmid (2 µg/well of a 6-well plate) and test nucleic acid sequences directed against either rat IL-1β (SEQ ID NO: 1 through SEQ ID NO: 6), human IL-1β (SEQ ID NO: 14 through SEQ ID NO: 17), rat TNFα (SEQ ID NO: 8 through SEQ ID NO: 13), human TNFα (SEQ ID NO: 18 through SEQ ID NO: 21), or control (SEQ ID NO: 7 and SEQ ID NO: 22). Forty-eight hours post-transfection, total cellular RNA was harvested from the cells and used to make cDNA by standard methods. The cDNAs were analyzed for target (IL-1β or TNFα) and reporter (GFP) gene expression levels using realtime PCR methods. The data was normalized for GFP expression levels to control for variation in transfection efficiency.

FIG. **5** shows the results of the rat IL-1β transfection studies related to each of the test nucleic acid sequence's ability to suppress rat IL-1β mRNA. As measured by realtime PCR, the CTRL sequence (SEQ ID NO: 7) suppressed IL-1β mRNA by 17.3%; SEQ ID NO: 1 suppressed IL-1β mRNA by 23.8%; SEQ ID NO: 2 by 80.1%; SEQ ID NO: 3 by 81.0%; SEQ ID NO: 4 by 85.8%; SEQ ID NO: 5 by 86.7 %; and SEQ ID NO: 6 by 0%. FIG. **6** shows the results of rat TNFα transfection studies related to each test nucleic acid sequence's ability to suppress rat TNFα mRNA (FIG **6A**) and protein (FIG **6B**). As measured by realtime PCR (FIG **6A**), the CTRL sequence (SEQ ID NO: 7) suppressed TNFα mRNA by 8.3%; SEQ ID NO: 8 suppressed TNFα mRNA by 74.8%; SEQ ID NO: 9 by 88.5%; SEQ ID NO: 10 by 97.3%; SEQ ID NO: 11 by 92.8%; SEQ ID NO: 12 by 89.9%; and SEQ ID NO: 13 by 96.8%. As measured by ELISA (FIG **6B**), the CTRL sequence (SEQ ID NO: 7) suppressed TNFα protein by 30.0%; SEQ ID NO: 8 suppressed TNFα protein by 79.8%; SEQ ID NO: 9 by 74.6%; SEQ ID NO: 10 by 91.5%; SEQ ID NO: 11 by 88.6%; SEQ ID NO: 12 by 82.1%; and SEQ ID NO: 13 by 94.1%.

The results described above regarding suppression of rat IL-1β mRNA demonstrate that the anti-rIL-1β nucleic acid sequences labeled SEQ ID NO: 2; SEQ ID NO: 3; SEQ ID NO: 4 and SEQ ID NO: 5 suppressed the target gene mRNA expression by about 80%. In relation to the anti-rIL-1β nucleic acid sequence represented by SEQ ID NO: 1, one should note that the cDNA used to construct pTracer-rIL-1β contained the open reading frame for mature (processed rIL-1β and did not contain the sequence targeted by this nucleic acid sequence. Therefore the data generated in this particular assay (using pTracer-rIL-1β) should be considered off-target gene silencing in relation to SEQ ID NO: 1. The CTRL nucleic acid sequence (SEQ ID NO: 7) also showed low levels of non-specific off-target mRNA suppression.

The results described above regarding suppression of rTNFα mRNA and protein demonstrate that the nucleic acid sequences represented by SEQ ID NO: 10 and SEQ ID NO: 13 were the best suppressors of rTNFα, each suppressing rTNFα mRNA and protein levels by greater than 90%.

In order to evaluate the ability of the anti-rIL-1β nucleic acid sequences and anti-rTNFα nucleic acid sequences to suppress endogenous protein after immune system stimulation, SEQ ID NO: 2; SEQ ID NO: 3; SEQ ID NO: 4 and SEQ ID NO: 5 as well as SEQ ID NO: 10 and SEQ ID NO: 13 (as well as off-target control sequences) were transfected individually into a rat macrophage cell line (NR8383). As a control, a different sample of NR8383 cells underwent a mock transfection procedure. Eight hours post-transfection, the cells were stimulated with LPS to induce rIL-1β and rTNFα protein expression or left untreated. The conditioned media then was collected 24 hours post-transfection and assayed by ELISA for rIL-1β and rTNFα protein levels. The level of rIL-1β and rTNFα protein observed in the mock transfected cells stimulated with LPS was set at 100%.

Regarding suppression of rIL-1β, FIG. **7** shows that four of the six tested nucleic acid sequences suppressed LPS-induced IL-1β protein production in the NR8383 cells (SEQ ID NO: 1, 2, 4, and 5). These results suggest that these nucleic acid sequences have the ability to target the endogenous rIL-1β mRNA for degradation and, as a result, suppress rIL-1β protein production. The nucleic acid sequence designated SEQ ID NO: 5 was found to be the most effective sequence and suppressed endogenous rIL-1β protein expression by 84.4%. Because the nucleic acid sequences designated SEQ ID NO: 3 and SEQ ID NO: 4 have complete sequence identity to both the rat and human IL-1β sequence, it is reasonable to expect that they both have the ability to suppress expression of this target gene in either species.

FIG. **8** shows that SEQ ID NO: 13 was the nucleic acid sequence most effective at suppressing endogenous rTNFα protein production after LPS stimulation of NR8383 cells. Indeed, SEQ ID NO: 13 was found to suppress rTNFα protein expression by greater than 80% while SEQ ID NO: 10 suppressed rTNFα protein expression by about 35%. These data suggest that SEQ ID NO: 13 is a potent suppressor of rTNFα production.

FIG. **9** shows the results of human IL-1β transfection studies related to each of the test nucleic acid sequence's ability to suppress hIL-1β mRNA and protein. As measured by realtime PCR, the CTRL sequence (SEQ ID NO: 7) suppressed hIL-1β mRNA by 8.7%; SEQ ID NO: 14 suppressed hIL-1β mRNA by 87.5%; SEQ ID NO: 15 suppressed hIL-1β mRNA by 96.9%; SEQ ID NO: 16 suppressed hIL-1β mRNA by 83.0%; and SEQ ID NO: 17 suppressed hIL-1β mRNA by 96.3% (FIG. **9A**). As measured by ELISA, protein expression was suppressed to the greatest extent by SEQ ID NO: 15 which suppressed hIL-1β protein by 70.7% (FIG. **9B****).**

FIG. **10** shows the results of the human TNFα transfection studies related to each of the test nucleic acid sequence's ability to suppress hTNFα mRNA and protein. As measured by realtime PCR, the CTRL sequence (SEQ ID NO: 7) did not suppress hTNFα mRNA; SEQ ID NO: 18 suppressed hTNFα mRNA by 77.2%; SEQ ID NO: 19 suppressed hTNFα mRNA by 70.5%; SEQ ID NO: 20 suppressed hTNFα mRNA by 85.3%; and SEQ ID NO: 21 suppressed hTNFα mRNA by 61.5% (FIG. **10A****).** As measured by ELISA, protein expression was suppressed to the greatest extent by SEQ ID NO: 20 which suppressed hTNFα protein by 92.2% (FIG. **10B****).**

In order to evaluate the ability of the anti-hIL-1β and anti-hTNFα nucleic acid sequences to suppress endogenous protein production after immune system stimulation, SEQ ID NO: 14; SEQ ID NO: 15; SEQ ID NO: 16; SEQ ID NO: 17; SEQ ID NO: 18; SEQ ID NO: 19; SEQ ID NO: 20 and SEQ ID NO: 21 as well as control SEQ ID NO: 7 were transfected individually into human primary monocytes isolated from peripheral blood. As a control, a different sample of human primary monocytes underwent a mock transfection procedure. Eight hours post-transfection, monocytes were stimulated with LPS to induce hIL-1β and hTNFq target gene expression. ELISAs were performed on conditioned media produced by the cells 24 hours post-LPS stimulation. The level of hIL-1β protein or TNFα protein observed in the mock transfected cells stimulated with LPS was set at 100%. As shown in FIG. **11A****,** all four nucleic acid sequences directed against hIL-1β suppressed hIL-1β protein production by about 20-40% with SEQ ID NO: 17 as the best suppressor with 40.2% suppression. As shown in FIG. **11** **B,** SEQ ID NO: 20 was the best suppressor of hTNFα protein with 64.0% suppression.

Preparation of rIL-1β, rTNFα and Control-2 shNA Sequences

Once effective nucleic acid sequences were identified using the above described co-transfection systems and experiments, a subset of these sequences were formatted into short hairpin nucleic acid ("shNA") structures. FIG. 12 shows the formatted structures and construction of anti-r/hIL-1β shNA (SEQ ID NO: 47; FIG. 12), anti-rTNFα shNA (SEQ ID NO: 48; FIG. 12B), and control shNA (SEQ ID NO: 49; FIG. 12C). The shNA sequences contain original effective nucleic acid sequences (boxed sequence shown in black), a nine nucleotide hairpin (underlined), the reverse complement of the nucleic acid sequence (italicized), and a Pol III terminator sequence (TTTTTT). Four partially overlapping synthesized oligonucleotides (-A, -B, -C, and -D) were used to assemble the shNAs. In two separate reactions, the A/C and B/D oligonucleotides for each shNA were annealed. Apal and EcoRI restriction enzyme-compatible ends also were included in the shNA structures for directional subcloning into a murine U6 promoter-containing shuttle vector (pSilencer1.0-U6; Ambion, Inc., Austin, TX; FIG. 13). The full-length shNAs (SEQ ID NOS: 47, 48, and 49) then were cloned into the ApaI/EcoRI-digested pSilencer vector using a three-way ligation reaction. The U6 promoter was used for constitutive high level expression of the nucleic acid sequences.

*Method for Construction of AAV-shNA Viral Production Plasmids*

The BamHI (675-1051) fragment (FIG.13) containing the shNA expression cassette (murine U6 promoter, shNA sequence, and Pol III terminator sequence) from each of the pSilencer shuttle vectors (rIL-1β, rTNFq and control-2) was recovered, blunted with T4 DNA polymerase, and subcloned into an AAV1/2 expression vector (deprAVETM; GeneDetect.com Ltd, Bradenton, FL). As shown in FIG. 14, in the final viral expression vector (used for virus production), the U6 promoter drives the expression of the shNA and the Woodchuck enhancer/chicken β-actin promoter drives the expression of the enhanced green fluorescent protein (WPRE/CAG-eGFP). These expression cassettes are flanked by viral inverted terminal repeats (ITR). The Woodchuck post-transcriptional regulatory element (WPRE) and the presence of a bovine growth hormone (BGH) polyadenlyation sequence ensure high transcription following cellular transduction.

Initially, cellular transduction of the viral constructs was evaluated in vitro. Individual wells of a 6-well plate containing HEK293 cells at 60-70% confluency were incubated for 48 hours with 1 x 1010 genomic particles of AAV-SEQ ID NO: 48; AAV- SEQ ID NO: 49; or were sham-treated. Cellular transductions were carried out in Gibco DMEM high glucose media containing 2% fetal bovine serum for two hours before the media was supplemented with additional fetal bovine serum (10% final concentration). FIG. 15 shows that AAV-SEQ ID NO: 48 treated cells were positive for GFP fluorescence (A) while sham treated cells (B) were not (AAV-SEQ ID NO: 49 cells not shown).

Next, in order to optimize in vivo studies using the above described technologies, the control virus, AAV-SEQ ID NO: 49, was used to evaluate in vivo cellular transduction. Transduction was measured in animals possessing sciatic nerve cryo-injuries by brightfield and fluorescent image analysis and in CCI animals by GFP fluorescence and GFP immunohistochemical ("IHC") staining.

Animals with cryolesions of their sciatic nerve were used to characterize viral transduction in vivo. Briefly, cryolesioning was performed under 2% isofluorane. The animal's right sciatic nerve was exposed and freed from adherent tissue at mid-thigh by separating the muscle (biceps femoris) by blunt dissection. Using a cotton swab tip dipped in liquid nitrogen, the sciatic nerve was touched for about two seconds to induce the injury. The lesioned sciatic nerve of animals then was injected with 5 µl PBS or 5 µL AAV-SEQ ID NO: 49 virus (2.0 x 1012 genomic viral particles/mL). After 10 days, animals were sacrificed and the sciatic nerve and dorsal root ganglion tissues from both the lesioned/injected side and the contralateral side were collected. Tissues were frozen immediately in OCT media and stored at -80oC until sectioning. Sections of sciatic nerve (SN) and dorsal root ganglion (DRG) were imaged using brightfield or fluorescence microscopy and scored blind as to the treatment condition for the presence or absence of GFP fluorescence. FIG. 16 (A-H) shows that both sciatic nerve and DRG tissue from animals receiving AAV-SEQ ID NO: 49 injections (2.0 x 1012 genomic viral particles/mL) in the sciatic nerve were positive for GFP while tissue from animals receiving PBS was not. Thus, this data indicates successful viral transduction of the tissues in vivo.

Viral transduction also was measured in vivo in CCI animals (as described above) by GFP fluorescence and GFP IHC staining. FIG. 17 shows GFP fluorescence results when the sciatic nerve of CCI animals was injected with 5 µL PBS (A) or 5 µL AAV-SEQ ID NO: 49 virus (B). Sciatic nerve tissues were harvested from animals at 10 and 21 days post-injection, preserved in OCT, and stored at -80oC until sectioning. Seven-micron thick sections from the OCT-preserved frozen samples were cut, protected from light, and evaluated under UV light/GFP filter. The results revealed that the suture material used to create the CCI injury fluoresced at the same wavelength as GFP preventing analysis of AAV transduction by direct fluorescence. Detection of AAV-SEQ ID NO: 49 transduction also was evaluated in vivo using GFP IHC staining. Again, the sciatic nerves of animals with CCI injuries were injected with 5 µL PBS (A) or 5 µL AAV-SEQ ID NO: 49 (B). The CCI sciatic nerve tissues were harvested, preserved in formalin, embedded in paraffin, and then cut into 5-micron thick sections. Sections were stained with hematoxylin and eosin (H&E), and evaluated. A rabbit polyclonal anti-GFP (ab290; Abcam Ltd, Cambridge UK) was used to evaluate GFP protein expression at the site of viral injection. As shown in FIG. 18, sections obtained from animals that received the virus (B) had slightly higher levels of GFP immunoreactivity than controls (A) suggesting viral transduction. The described data suggests that the shNA sequences created as described herein can be introduced into cells in vivo using viral transduction methods.

An alternative behavioral model was chosen to evaluate the efficacy of pro-inflammatory cytokine suppression on a pain-associated behavior (tactile allodynia) following intrathecal (i.t.) administration of stabilized siRNA. The spinal nerve ligation model (SNL; Kim and Chung, 1992, Pain 50:355-363), is also a commonly used model of neuropathic pain. Male Sprague Dawley rats (275-300 grams) were implanted with chronic i.t. catheters according to the procedure originally described by Yaksh and Rudy (1976, Physiol Behav 1:1031-1036). Briefly, rats were anesthetized with 2% isoflurane and the back of the head and neck was shaved. The animals were then placed in a stereotaxic head frame with the head flexed forward. A midline incision was made on the skull exposing the cisternal membrane. The membrane was opened with a stab blade and an 8.5 cm polyethylene (PE-10) catheter was then inserted through the cisternal opening, and passed caudally into the i.t. space at the L1-L3 spinal segments. The end of the catheter was tunneled through the subcutaneous (s.c.) space over the frontal bones, flushed with 10 µL saline, plugged with a short length of wire, and left exposed for future administration of test and control agents. The skin incision was closed using 3.0 USP braided silk suture. The rats were given 5 mL of lactated Ringer's solution s.c. and allowed to recover under a heat lamp. Rats showing motor weakness or signs of paresis upon recovery from anesthesia were sacrificed and not used for compound evaluation.

Four days post-surgery (Day 4), animals began a dosing protocol in which they were injected with the designated agent through the exposed catheter. Each injection was in a 20 µL volume followed by a 10 µL flush with physiologic saline. Three injections were made in each animal on Day 4, about 6 hours apart. A fourth injection was performed on Day 7 and a fifth injection on Day 11. TNF□ siRNA (SEQ NO: 13) and control siRNA (SEQ NO: 7) containing enhanced stabilization chemistries (siStable®; Dharmacon Inc.; Lafayette, CO) were used for these in vivo studies. Ten micrograms of siRNA in a volume of 10 01 was complexed with 10 µL of ExGen500 polyethylenimine (PEI) for about 10 minutes according to the recommendations of the manufacturer (Fermentas Life Sciences; Burlington, Ontario, Canada). PEI only vehicle injections consisted of 10 µL 5% dextrose complexed with 10 µL of the ExGen500 reagent.

All animals received a modified SNL injury (ligation and transection of spinal nerves L5 and L6) according to the method of Tsuda et al. (2004, Glia, 89:89-95 ) on the left side on Day 7 post-catheter implant.

Animals were tested for tactile allodynia (pain-associated behavior) using the Dixon up-down method (Dixon, 1980 Ann Rev Pharmacol Toxicol 20:441-462 and Chaplan et al., 1994 J Neurosci Methods 53:55-63) for determination of 50% probability withdrawal thresholds at various timepoints in this study. Pre-SNL injury baseline testing was performed to evaluate allodynia induced by catheter placement and siRNA treatment on Days 3 and 7, respectively. Animals did not enter the study if withdrawal threshold was below 13g on either foot prior to the first intrathecal injection. Post-SNL injury testing was also performed on Days 11 and 14 to evaluate efficacy of siRNA administration on the allodynic behavior.

The results of the tactile allodynia testing are summarized in Table 3 below. Day 3 test results indicate that chronic implant of the catheter did not alter pre-SNL paw withdrawal thresholds. Day 7 testing showed that while there was a tendency for the siRNA-treated animals to have lower thresholds than either the PEI or the saline groups after 4 days after the three IT injections, but before the nerve injury, this tendency was not significant. The largest difference on Day 7 was between the TNFα siRNA- and saline-treated rats (p=0.34; unpaired t-test). Treatment efficacy testing conducted on Day 11 (post-SNL injury) indicated that the TNFα siRNA treatment significantly improved tactile allodynia compared to PEI (p=0.0013), saline (p=0.0001), and control siRNA (p=0.0471). The results of the Day 14 tests showed a trend for reduced allodynia in the TNFα siRNA-treated animals relative to saline and PEI only controls, but did not reach the level of statistical significance (p=0.0586 and p=0.0667, respectively). The inability to achieve conventional statistical significance (p=0.05) may have been due to the lower dosing regimen used later in this study. Overall, this study demonstrated that siRNA directed against TNFα can reduce a pain-associated behavior (allodynia) when administered into the intrathecal space of SNL-injured animals.

**Table 3.**

| **Treatment Agent** | **# Animals** | **Day 3** | **Day 7** | **Day 11** | **Day 14** |
|---|---|---|---|---|---|
| TNFα siRNA (SEO ID NO: 13) | 8 | 15.0 | 10.98±1.6 | 5.65±0.7 | 4.66±1.1 |
| Control siRNA (SEQ ID NO: 7) | 8 | 15.0 | 11.29+1.7 | 3.6±0.7 | 2.65±0.5 |
| PEI | 7 | 15.0 | 13.26±1.4 | 2.31 ±0.5 | 2.51±0.4 |
| Saline | 8 | 15.0 | 13.83±1.0 | 1.94±0.2 | 2.38±0.6 |

### Example 1

To identify if the above identified nucleic acid sequences suppress human type I and type II IL-1β receptors as well as human type I and type II TNFα receptors, an *in vitro* co-transfection assay system is developed. Type I and type II human IL-1β receptor gene sequences ("hIL-1β RI" and "hIL-1β RII" respectively) as well as type I and type II human TNFα receptor gene sequences ("hTNFα RI" and "hTNFα RII" respectively) are subcloned into pTracer^{™}-CMV2 (Invitrogen, Corp., Carlsbad, CA) to generate pTracer-hIL-1β RI, pTracer-hIL-1β RII pTracer-hTNFα RI and pTracer-hTNFα RI. The recombinant plasmid also includes a GFP-Zeocin reporter gene for transfection efficiency normalization. The CMV promoter directs constitutive expression of the target genes (hIL-1β RI, hIL-1β RII, hTNFα RI and hTNFα RII) while the EF1 promoter directs constitutive expression of the GFP-Zeocin reporter gene.

The generated recombinant plasmids are used to facilitate screening of nucleic acid sequences by co-transfection into a eukaryotic cell line. Specifically, HEK293 or HeLa cell cultures at 60-70% confluency are co-transfected with the appropriate target plasmid (2 µg/well of a 6-well plate) and test nucleic acid sequences are directed against either hIL-1β RI (SEQ ID NO: 23 through SEQ ID NO: 28), hIL-1β RII (SEQ ID NO: 29 through SEQ ID NO: 34), hTNFα RI (SEQ ID NO: 35 through SEQ ID NO: 40), hTNFα RII (SEQ ID NO: 41 through SEQ ID NO: 46), or control (SEQ ID NO: 7 and SEQ ID NO: 22). Forty-eight hours post-transfection, total cellular RNA is harvested from the cells and used to make cDNA by standard methods. The cDNAs are analyzed for target (hIL-1β RI, hIL-1β RII, hTNFα RI and hTNFα RII) and reporter (GFP) gene expression levels using realtime PCR methods. The data is normalized for GFP expression levels to control for variation in transfection efficiency.

Using realtime PCR to measure the mRNA of interest, the results of this study will show that the nucleic acid sequences directed against hIL-1β RI, hIL-1β RII, hTNFα RI and hTNFα RII inhibit gene expression relative to controls. This data will suggest that the nucleic acid sequences identified against these receptor types are effective at inhibiting production of these receptor types.

In order to evaluate the ability of the same nucleic acid sequences to suppress endogenous protein production after immune system stimulation, SEQ ID NO: 23 through SEQ ID NO: 46, as well as control SEQ ID NO: 7 are transfected individually into human primary monocytes isolated from peripheral blood. As a control, a different sample of human primary monocytes undergoes a mock transfection procedure. Eight hours post-transfection, monocytes are stimulated with LPS to increase hIL-1β RI, hIL-1β RII, hTNFα RI and hTNFα RII, target gene expression. The level of hIL-1β RI, hIL-1β RII, hTNFα RI and hTNFα RII mRNA observed in the mock transfected cells stimulated with LPS is set at 100%. The results of this study will show that the nucleic acid sequences effectively suppress pro-inflammatory cytokine receptor gene expression after LPS administration.

### Example 2

A study is undertaken to evaluate the effectiveness of locally administering the shNA sequences identified above (SEQ ID NO: 47-49) for the treatment of pain. As a baseline measure, naïve animals are tested for pain sensitivity. Each animal is placed in the clear plastic chamber of a plantar analgesia instrument (Stoelting Co., Wood Dale, IL) and allowed to acclimate for 15 minutes. After the acclimation period, a radiant (heat) beam source stimulus is applied to a hind paw of each animal. The heat source device is set at an intensity of 50 as recommended by the manufacturer, and a maximum latency period of 15 seconds is set to prevent tissue damage. If a paw withdrawal occurs within the 15 second period, an automated control interrupts both the stimulus and timer, turning off the radiant beam and recording the latency of time to paw withdrawal.

The following day, half of the animals undergo chronic constriction injury ("CCI") and half of the animals undergo sham surgery. Each animal is anesthetized by intraperitoneal ("i.p.") injection of sodium pentobarbital at a dose of 60mg/kg body weight. The animal's common sciatic nerve (ipsilateral to the hindpaw tested on the previous day) is exposed and freed from adherent tissue at mid-thigh by separating the muscle (biceps femoris) by blunt dissection. In CCI animals, four loose ligatures are placed 1 mm apart, using chromic gut (4-0 absorbable suture, Jorgensen Laboratories Inc., Loveland, CO). No ligatures are placed on the sciatic nerve of sham surgery animals.

Behavioral measures are recorded 2, 4 and 6 days following CCI or sham surgery to verify the surgical efficacy of inducing acute and neuropathic pain states. At 2 days post-surgery, all animals are sensitized to the radiant beam source, showing reduced paw withdrawal latencies. At 4 and 6 days post surgery, the sham-surgery animals begin to trend towards pre-surgery paw withdrawal latencies suggesting that pain is receding as the wound associated with the surgery heals. Importantly, these animals do not return entirely to pre-surgery paw withdrawal levels by day 6 indicating some continued pain sensitivity. In contrast to the sham surgery animals, CCI animals remain sensitized to the radiant beam source, suggesting a neuropathic pain state.

On day 7, CCI and sham-surgery animals are divided into five groups. Group one receives intrathecal ("i.t.") administration of 10 µL PBS. Group two receives i.t. administration of 10 µL AAV-SEQ ID NO:49 (CTRL). Group three receives i.t. administration of 5 µL AAV-SEQ ID NO: 47 (anti-r/hIL-1β) and 5 µL AAV-SEQ ID NO: 49 (CTRL). Group four receives i.t. administration of 5 µL AAV-SEQ ID NO: 48 (anti-rTNFα) and 5 µL AAV-SEQ ID NO: 49 (CTRL). Group five receives i.t. administration of 5 µl AAV-SEQ ID NO: 47 (anti-r/hIL-1β) and 5 µl AAV-SEQ ID NO: 48 (anti-rTNFα). Animals then are tested for paw withdrawal latencies every other day for 4 days. After testing on the fourth day, animals receive the same injection received on day 7 and are tested every other day for 4 more days. For purposes of behavioral testing, groups one and two are referred to collectively as the control group.

The behavioral data demonstrate that the i.t. administration of either AAV-SEQ ID NO: 47 or AAV-SEQ ID NO: 48 increase paw withdrawal latencies relative to controls in both CCI and sham-surgery animals. Animals receiving AAV-SEQ ID NO: 47 and AAV-SEQ ID NO: 48 show a greater increase in paw withdrawal latencies than those receiving AAV-SEQ ID NO: 47 or AAV-SEQ ID NO: 48 alone. Control CCI animals remain sensitized to the radiant beam source throughout the testing period suggesting the on-going presence of a neuropathic pain state. Control sham-surgery animals slowly increase their paw withdrawal latencies over the behavioral testing days further indicating the continued abatement of heightened pain sensitivity during an acute pain state.

Following behavioral testing, animals are anesthetized by i.p. injection of sodium pentobarbital at a dose of 100 mg/kg body weight and transcardially perfused with 0.9% phosphate buffered saline followed by 10% phosphate buffered formalin. Spinal cords are removed and the lumbar region is preserved in formalin, embedded in paraffin, and cut into 5 micron thick sections. Sections are then stained with hematoxylin and eosin (H&E) and evaluated. A rabbit polyclonal anti-GFP antibody (ab290; Abcam Ltd, Cambridge UK) is used to evaluate viral transduction. *In vivo* immunohistochemical (IHC) staining with an anti-GFP antibody demonstrates a high level of viral transduction in all animals receiving an AAV viral construct as compared to animals receiving the PBS control.

### Example 3

Example 2 shows that i.t. administration of shNAs that suppress the expression of amino acid sequences that yield pro-inflammatory cytokines can reduce pain sensitivity caused by pro-inflammatory cytokines effectively. A second experiment is carried out that mimics the first experiment except that drug and vehicle administration is made in the perispinal region of the lumbar region of the spinal cord rather than in the i.t. space. The data will demonstrate that the perispinal administration of the drugs generates comparable results as their i.t. administration suggesting that the perispinal region provides an alternatively effective administration route.

### Example 4

A double-blind, placebo controlled study will be conducted over a three-week period. A total of 270 subjects, all presenting for treatment of either acute pain or neuropathic pain related to sciatica, are chosen for the study. The patients range in age from 35-65 years old.

An initial assessment of the symptoms of each patient is conducted when the patients initially present for treatment. The treating physician rates the severity of the acute or neuropathic pain on a 4-point scale (1: mild; 2: moderate; 3: strong; 4: severe).

The 270 subjects chosen for the study are separated into nine separate groups of 30. The severity of the symptoms between the groups are comparable and patients with acute versus neuropathic pain are distributed evenly among the groups. No other medications are taken by the patients during the assessment period.

According to group assignment, patients receive perispinal administration of 10 mg of stabilized nucleic acid sequences every 7 days according to the following combinations: Group 1: 10 mg SEQ ID NO: 7 (CTRL); Group 2: 5 mg of SEQ ID NO: 17 (anti-hIL-1β) and 5 mg of SEQ ID NO: 7 (CTRL); Group 3: 5 mg of SEQ ID NO: 20 (anti-hTNFα) and 5 mg AAV-SEQ ID NO: 7 (CTRL); Group 4: 5 mg of SEQ ID NO: 17 (anti-hIL-1 β) and 5 mg SEQ ID NO: 20 (anti-hTNFα); Group 5: 5 mg of SEQ ID NO: 23 (anti-hIL-1β RI) and 5 mg of SEQ ID NO: 7 (CTRL); Group 6: 5 mg of SEQ ID NO: 29 (anti-hIL-1β RII) and 5 mg of SEQ ID NO: 7 (CTRL); Group 7: 5 mg of SEQ ID NO: 35 (anti-hTNFα RI) and 5 mg of SEQ ID NO: 7 (CTRL); Group 8: 5 mg of SEQ ID NO: 41 (anti-hTNFα RII) and 5 mg of SEQ ID NO: 7 (CTRL); and Group 9: 5 mg of SEQ ID NO: 23 (anti-hIL-1β RI) and 5 mg of SEQ ID NO: 35 (anti-hTNFα RII).

During the 3 week evaluation period, patients self-evaluate the severity of their symptoms related to acute or neuropathic sciatic pain using the same 4-point scale (1: mild; 2: moderate; 3: strong; 4: severe) each morning. Patients also note the presence and severity of adverse effects of the drug administration on the 4-point scale. In addition to the initial assessment by the treating physician, patients are evaluated at the end of each week by the treating physician.

An improvement in acute and neuropathic sciatic pain will be observed in the treated subjects over the controls upon completion of the study. Moreover, the patients receiving SEQ ID NO: 17 (anti- hIL-1β) and SEQ ID NO: 20 (anti-hTNFα) are anticipated to show greater improvement than those receiving either sequence alone. Similarly, patients receiving SEQ ID NO: 23 (anti-hIL-1β RI) and SEQ ID NO: 35 (anti-hTNFα RI) show greater improvement than those receiving either sequence alone. This study will demonstrate the efficacy of the nucleic acid sequences of the present invention in treating acute or neuropathic sciatic pain. Thus, this study will demonstrate that the local administration of nucleic acid sequences directed against IL-1β and TNFα expressions as well as those sequences directed against expression of their receptors provides an effective treatment for acute and neuropathic sciatic pain. It is anticipated that the treatment will be well-tolerated by patients.

The siNA sequences and molecules of the present invention can be manipulated to enhance their uptake into the RISC complex. Specifically, manipulating the 3 prime terminal nucleotide of the sense strand can be highly advantageous. Preferential entry of the guide, or antisense, strand into RISC can be achieved by introducing 3 prime mismatches in the sense strand while maintaining perfect base pairing (of the antisense strand and the intended mRNA target) at the 5 prime terminus of the antisense strand. This maximizes entry of the antisense strand into the RISC complex, while also reducing potential off-target inhibition by the sense strand.

Physical methods to introduce nucleic acid molecules and/or their carriers (i.e. vectors) into eukaryotic cells are known in the art. Some of these methods include, without limitation, calcium phosphate or calcium chloride co-precipitation, DEAE-dextran-mediated transfection, cationic lipid-mediated transfection, lipofection, protoplast fusion, particle bombardment, microinjection, liposome fusion, electroporation, biolistics and other suitable methods found in, for example, Sambrook, et al. (Molecular Cloning: A Laboratory Manual, 2nd, ed, Cold Spring Harbor Laboratory, Cold Spring Harbor Laboratory Press, Cold Spring Harbor, N.Y., 1989), and other laboratory manuals. Further, biological methods to introduce nucleic acid molecules into a cell include the use of viral vectors. For mammalian gene therapy, viral vectors, and especially retroviral vectors, have become the most widely used method for inserting genes into mammalian cells. Other viral vectors can be derived from poxviruses, herpes simplex virus I, adenoviruses and adeno-associated viruses, and the like (see, for example, Boenisch et al., USPN 5,350,674 and Wilson et al., USPN 5,585,362

The nucleic acid molecules/vehicle combinations can be locally delivered by catheter and drug pump systems, delivered by direct local injection or through the use of polymers and/or drug-eluting stents as described in co-pending U.S. Patent Application No. 10/972,157 which is incorporated by reference herein. In one embodiment, a "controlled administration system" including a direct and local administration system can be used. A controlled administration system can be a depot or a pump system, such as, without limitation, an osmotic pump or an infusion pump. An infusion pump can be implantable and can be, without limitation, a programmable pump, a fixed rate pump, and the like. A catheter can be operably connected to the pump and configured to deliver agents of the present invention to a target tissue region of a subject. A controlled administration system can be a pharmaceutical depot (a pharmaceutical delivery composition) such as, without limitation, a capsule, a microsphere, a particle, a gel, a coating, a matrix, a wafer, a pill, and the like. A depot can comprise a biopolymer. The biopolymer can be a sustained-release biopolymer. The depot can be deposited at or near, generally in close proximity, to a target site. Embodiments of the present invention also can be delivered through the use of liposomes, polyethyleneimine, by iontophoresis, or by incorporation into other vehicles, such as biodegradable nanocapsules.

The present invention also includes systems and kits. One embodiment of the systems of the present invention comprises nucleic acid molecules comprising one or more sequences that suppress the expression of amino acid sequences encoding for pro-inflammatory cytokines and/or pro-inflammatory cytokine receptors wherein the sequences are selected from the group consisting of SEQ ID NO: 14; SEQ ID NO: 15; SEQ ID NO: 16; SEQ ID NO: 17; SEQ ID NO: 18; SEQ ID NO: 19; SEQ ID NO: 20; SEQ ID NO: 21; SEQ ID NO: 23; SEQ ID NO: 24; SEQ ID NO: 25; SEQ ID NO: 26; SEQ ID NO: 27; SEQ ID NO: 28; SEQ ID NO: 29; SEQ ID NO: 30; SEQ ID NO: 31; SEQ ID NO: 32; SEQ ID NO: 33; SEQ ID NO: 34; SEQ ID NO: 35; SEQ ID NO: 36; SEQ ID NO: 37; SEQ ID NO: 38; SEQ ID NO: 39; SEQ ID NO: 40; SEQ ID NO: 41; SEQ ID NO: 42; SEQ ID NO: 43; SEQ ID NO: 44; SEQ ID NO: 45; SEQ ID NO: 46 and combinations thereof and wherein said system is used to treat pain locally.

Another embodiment of the systems of the present invention comprises nucleic acid molecules comprising one or more sequences that suppress the expression of amino acid sequences encoding for pro-inflammatory cytokines and/or pro-inflammatory cytokine receptors wherein the sequences are locally administered through a catheter and drug pump to treat pain. The catheter and drug pump can be one of the types previously described.

The present invention also includes kits. In one embodiment, the kits of the present invention comprise injectable forms of the nucleic acid molecules of the present invention. In another embodiment, a kit of the present invention can contain one or more of the following in a package or container: (1) one or more nucleic acid molecules of the present invention; (2) one or more pharmaceutically acceptable adjuvants or excipients; (3) one or more vehicles for administration, such as one or more syringes; (4) one or more additional bioactive agents for concurrent or sequential administration; (5) instructions for administration; and/or (6) a catheter and drug pump. Embodiments in which two or more of components (1) - (6) are found in the same container can also be used.

When a kit is supplied, the different components of the compositions included can be packaged in separate containers and admixed immediately before use. Such packaging of the components separately can permit long-term storage without losing the active components' functions. When more than one bioactive agent is included in a particular kit, the bioactive agents may be (1) packaged separately and admixed separately with appropriate (similar or different) vehicles immediately before use, (2) packaged together and admixed together immediately before use or (3) packaged separately and admixed together immediately before use. If the chosen compounds will remain stable after admixture, however, the admixture need not occur immediately before use but can occur at a time before use, including in one example, minutes, hours, days, months or years before use or in another embodiment at the time of manufacture.

The compositions included in particular kits of the present invention can be supplied in containers of any sort such that the life of the different components are preserved and are not adsorbed or altered by the materials of the container. For example, sealed glass ampules can contain lyophilized agents or variants or derivatives thereof or other bioactive agents, or buffers that have been packaged under a neutral, non-reacting gas, such as, without limitation, nitrogen. Ampules can consist of any suitable material, such as, without limitation, glass, organic polymers, such as, polycarbonate, polystyrene, etc., ceramic, metal or any other material typically employed to hold similar reagents. Other examples of suitable containers include, without limitation, simple bottles that may be fabricated from similar substances as ampules, and envelopes, that can comprise foil-lined interiors, such as aluminum or an alloy. Other containers include, without limitation, test tubes, vials, flasks, bottles, syringes, or the like. Containers can have one or more sterile access ports, such as a bottle having a stopper that can be pierced by a hypodermic injection needle. Other containers may have two compartments that are separated by a readily removable membrane that upon removal permits the components to be mixed. Removable membranes may be, without limitation, glass, plastic, rubber, etc.

As stated earlier, kits can also be supplied with instructional materials. Instructions may be printed on paper or other substrate, and/or may be supplied as an electronic-readable medium, such as a floppy disc, CD-ROM, DVD-ROM, Zip disc, videotape, audiotape, flash memory device, etc. Detailed instructions may not be physically associated with the kit; instead, a user may be directed to an internet web site specified by the manufacturer or distributor of the kit, or supplied as electronic mail.

As should be understood, the exact formulation, route of administration, and dosage should generally be determined by the attending physician in view of the patient's condition. Dosage amount and interval can be adjusted individually to provide appropriate levels of nucleic acid molecules which are sufficient to maintain therapeutic effect.

It is to be understood that the present invention is not limited to the particular embodiments, materials, and examples described herein, as these can vary. For example, the nucleic acid molecules of the present invention can be created in a variety of formats and lengths. Indeed, those skilled in the art will recognize that the most important attribute of the nucleic acid molecules of the present invention is their ability to complementarily bind to the mRNA sequences of interest to reduce the mRNA stability and/or the translation rate of these sequences. The phrase "complementarily bind" as used herein, refers to the abilities of the nucleic acid molecules to form hydrogen bond(s) with mRNA sequences by either traditional Watson-Crick pairing or other non-traditional types. While binding due to 100% complementarity is preferred, complementarity as low as 50-75% also is useful in accordance with the methods of the present invention.

The nucleic acid molecules of the present invention can include any type of nucleic acid molecule capable of mediating RNA interference, such as, without limitation, short interfering nucleic acid (siNA), short hairpin nucleic acid (siNA), short interfering RNA (siRNA), short hairpin RNA (shRNA), micro-RNA (miRNA), and double-stranded RNA (dsRNA). The nucleic acid molecules of the present invention also include similar DNA sequences. Further, these nucleic acid molecules can contain unmodified or modified nucleotides. Modified nucleotides refer to nucleotides which contain a modification in the chemical structure of a nucleotide base, sugar and/or phosphate. Such modifications can be made to improve the stability and/or efficacy of nucleic acid molecules and are described in patents and publications such as Beigelman et al., U.S. Pat. No. 6,617,438, Sproat, U.S. Pat. No. 5,334,711; Beigelman et al., International PCT publication No. WO 97/26270; Beigelman et al., U.S. Pat. No. 5,716,824; Usman et al., U.S. Pat. No. 5,627,053; Woolf et al., International PCT Publication No. WO 98/13526; Thompson et al., U.S. Ser. No. 60/082,404 which was filed on Apr. 20, 1998; Eckstein et al., International Publication PCT No. WO 92/07065; McSwiggen et al., WO03/070897 Usman et al. International Publication PCT No. WO 93/15187; Beigelman et al., 1995, J. Biol. Chem., 270, 25702; Usman and Cedergren, 1992, TIBS. 17, 34; Usman et al., 1994, Nucleic Acids Symp. Ser. 31, 163; Burgin et al., 1996, Biochemistry, 35, 14090; Perrault et al. Nature, 1990, 344, 565-568; Pieken et al. Science, 1991, 253, 314-317; Usman and Cedergren, Trends in Biochem. Sci., 1992, 17, 334-339; Karpeisky et al., 1998, Tetrahedron Lett., 39, 1131; Earnshaw and Gait, 1998, Biopolymers (Nucleic Acid Sciences), 48, 39-55; Verma and Eckstein, 1998, Annu. Rev. Biochem., 67, 99-134; Burlina et al., 1997, Bioorg. Med. Chem., 5, 1999-2010; Limbach et al., 1994, Nucleic Acids Res. 22, 2183; and Burgin et al., 1996, Biochemistry, 35, 14090. Such patents and publications describe general methods and strategies to modify nucleic acid molecules, In view of such teachings, similar modifications can be used to modify the nucleic acid molecules of the instant invention. The nucleic acid molecules of the present invention can include all unmodified nucleotides, all modified nucleotides or a mixture of unmodified and modified nucleotides.

The nucleic acid molecules of the present invention also can be created in a variety of lengths. Again, any length can be used so long as the nucleic acid molecules of the present invention complementarily bind to the mRNA sequences of interest to reduce the mRNA stability and/or the translation rate of these sequences. In one embodiment of the methods and nucleic acid molecules of the present invention, the molecules are about 19-21 base pairs. In another embodiment of the methods and nucleic acid molecules of the present invention, the molecules are about 25-27 base pairs. Again, however, these lengths are non-limiting examples.

It also is to be understood that the terminology used herein is used for the purpose of describing particular embodiments only, and is not intended to limit the scope of the present invention. It must be noted that as used herein and in the appended claims, the singular forms "a," "an," and "the" include the plural reference unless the context clearly dictates otherwise. Thus, for example, a reference to "a sequence" or "a shNA" is a reference to one or more sequences or shNAs and includes equivalents thereof known to those skilled in the art and so forth.

Unless defined otherwise, all technical terms used herein have the same meanings as commonly understood by one of ordinary skill in the art to which this invention belongs.

## Claims

1. The use of nucleic acid molecules comprising sequences that suppress the expression of amino acid sequences encoding for inflammatory response proteins in the manufacture of a medicament for the therapeutic treatment of pain by local administration, wherein said inflammatory response proteins are pro-inflammatory cytokines selected from the group consisting of interleukin-1 beta ("IL-1β") and tumor necrosis factor alpha ("TNFα") or are pro-inflammatory cytokine receptors selected from the group consisting of type I IL-1β receptors, type II IL-1β receptors, type I TNFα receptors and type II TNFα receptors, **characterised in that**
when said pro-inflammatory cytokine isIL-1β said nucleic acid molecules comprise mammalian nucleotide sequences selected from the group consisting of SEQ ID NO: 14; SEQ ID NO: 15; SEQ ID NO: 16; SEQ ID NO: 17; SEQ ID NO: 14 in shNA format; SEQ ID NO: 15 in shNA format; SEQ ID NO: 16 in shNA format; SEQ ID NO: 17 is shNA format and combinations thereof;
or when said pro-inflammatory cytokine is TNFα said nucleic acid molecules are mammalian nucleotide sequences selected from the group consisting of SEQ ID NO-18; SEQ ID NO: 19; SEQ ID NO: 20; SEQ ID NO 21; SEQ ID NO: 18 in shNA format; SEQ ID NO: 19 in shNA format; SEQ ID NO: 20 in shNA format; SEQ ID NO: 21 in shNA format and combinations thereof;
or when said pro-inflammatory cytokine receptor is a type I IL-1 β receptor said nucleic acid molecules are mammalian nucleotide sequences selected from the group consisting of SEQ ID NO: 23; SEQ ID NO: 24; SEQ ID NO: 25; SEQ ID NO: 26; SEQ ID NO: 27; SEQ ID NO: 28; SEQ ID NO: 23 in shNA format; SEQ ID NO: 24 is shNA format; SEQ ID NO: 25 in shNA format; SEQ ID NO: 26 in shNA format; SEQ ID NO: 27 in shNA format; SEQ ID NO: 28 in shNA format and combinations thereof;
or when wherein said pro-inflammatory cytokinereceptor is a type II IL-1β receptor said nucleic acid molecules are mammalian nucleotide sequences selected from the group consisting of SEQ ID NO: 29; SEQ ID NO: 30; SEQ ID NO: 31, SEQ ID NO: 32; SEQ ID NO: 33; SEQ ID NO: 34; SEQ ID NO: 29 in shNA
format; SEQ ID NO: 30 in shNA format; SEQ ID NO: 31 in shNA format; SEQ ID NO: 32 in shNA format; SEQ ID NO: 33 in shNA format; SEQ ID NO: 34 in shNA format and combinations thereof;
or when said pro-inflammatory cytokine receptor is a type I TNFα receptor said nucleic acid molecules are mammalian nucleotide sequences selected from the group consisting of SEQ ID NO: 35; SEQ ID NO: 36; SEQ ID NO: 37; SEQ ID NO: 38; SEQ ID NO: 39; SEQ ID NO: 40; SEQ ID NO: 35 in shNA format; SEQ ID NO; 36 in shNA format; SEQ ID NO; 37 in shNA format; SEQ ID NO: 38 in shNA format; SEQ ID NO: 39 in shNA format; SEQ ID NO:40 in shNA format and combinations thereof;
or when said pro-inflammatory cytokine receptor is a type II TNFα receptor said nucleic acid molecules are mammalian nucleotide sequences selected from the group consisting of SEQ ID NO: 41; SEQ ID NO: 42; SEQ ID NO: 43; SEQ ID NO: 44; SEQ ID NO: 45; SEQ ID NO: 46; SEQ ID NO: 41 in shNA format; SEQ ID NO: 42 in shNA format; SEQ ID NO: 43 in shNA format; SEQ ID NO:44 in shNA format; SEQ ID NO: 45 in shNA format; SEQ ID NO: 46 in shNA format and combinations thereof.

2. The use according to claim 1, wherein said pain is acute or neuropathic.

3. The use according to claim 1, wherein said pain is sciatica.

4. The use according to claim 1, wherein said nucleic acid molecules are to be administered locally to the perispinal region of the lumbar region of a spinal cord or are to be administered locally to the intrathecal space of the lumbar region of a spinal cord.

5. The use according to claim 1, wherein said nucleic acid molecules are to be administered locally from one of the administration routes selected from the group consisting of a catheter and drug pump, one or more local injections and polymer release.

6. The nucleic acid molecule, wherein said nucleotide sequence in a mammalian sequence selected from the group consisting of SEQ ID NO: 14; SEQ ID NO: 15; SEQ ID NO; 16; SEQ ID NO: 17; SEQ ID NO: 14 in shNA format; SEQ ID NO: 15 in shNA format; SEQ ID NO: 16 in shNA format ; and SEQ ID NO: 17 in shNA format, and said nucleotide sequence is a mammalian sequence selected from the group consisting of SEQ ID NO: 18; SEQ ID NO: 19; SEQ ID NO: 20; SEQ ID NO: 21; SEQ ID NO: 18 in shNA format; SEQ ID NO: 19 in shNA format; SEQ ID NO: 20 in shNA format; and SEQ ID NO: 21 in shNA format.

7. The nucleic acid molecule, wherein said nucleic acid molecules are
mammalian nucleotide sequences
selected from the group consisting of SEQ ID NO: 23; SEQ ID NO: 24; SEQ ID NO: 25; SEQ ID NO: 26; SEQ ID NO: 27; SEQ ID NO: 28; SEQ ID NO: 23 in shNA format; SEQ ID NO: 24 in shNA format; SEQ ID NO: 25 in shNA format; SEQ ID NO:26 in shNA format; SEQ ID NO: 27 in shNA format; and SEQ ID NO: 28 in shNA format.

8. The nucleic acid molecule, wherein said nucleic acid molecules are mammalian nucleotide sequences selected from the group consisting of SEQ ID NO: 29; SEQ ID NO: 30; SEQ ID NO: 31; SEQ ID NO: 32; SEQ ID NO: 33; SEQ ID NO: 34; SEQ ID NO: 29 in shNA format; SEQ ID NO: 30 in shNA format; SEQ ID NO: 31 in shNA format; SEQ ID NO: 32 in shNA format; SEQ ID NO: 33 in shNA format; and SEQ ID NO: 34 in shNA format.

9. The nucleic acid molecule, wherein said nucleic acid molecules are mammalian nucleotide sequence selected from the group consisting of SEQ ID NO: 35; SEQ ID NO: 36; SEQ ID NO: 37; SEQ ID NO: 38; SEQ ID NO: 39; SEQ ID NO: 40; SEQ ID NO: 35 in shNA format; SEQ ID NO: 36 in shNA format; SEQ ID NO: 37 in shNA format; SEQ ID NO: 38 in shNA format; SEQ ID NO: 39 in shNA format; and SEQ ID NO: 40 in shNA format.

10. The nucleic acid molecule, wherein said nucleic acid molecules are mammalian nucleotide sequences selected from the group consisting of SEQ ID NO: 41; SEQ ID NO: 42; SEQ ID NO: 43; SEQ ID NO: 44; SEQ ID NO: 45; SEQ ID NO: 46; SEQ ID NO: 41 in shNA format; SEQ ID NO: 42 in shNA format; SEQ ID NO: 43 in shNA format; SEQ rD NO: 44 in shNA format; SEQ ID NO: 45 in shNA format; and SEQ ID NO: 46 in shNA format.

11. Use of a kit comprising: nucleic acid molecules comprising one or more sequences that suppress the expression of amino acid sequences encoding for inflammatory response proteins in the manufacture of a medicament for the therapeutic treatment of pain, and an administration form used to locally deliver said nucleic acid molecules, wherein said inflammatory response proteins are pro-inflammatory cytokines selected from the group consisting of interleukin-1 beta ("IL-1β") and tumor necrosis factor alpha ("TNFα") or are pro-inflammatory cytokine receptors selected from the group consisting of type I IL-1β receptors, type II IL-1β receptors, type I TNFα receptors and type II TNFα receptors, **characterised in that**
when said pro-inflammatory cytokine is IL-1β said nucleic acid molecules comprise mammalian nucleotide sequences selected from the group consisting of SEQ ID NO: 14; SEQ ID NO: 15; SEQ ID NO: 16; SEQ ID NO: 17; SEQ ID NO: 14 in shNA format; SEQ ID NO: 15 in shNA format; SEQ ID NO: 16 in shNA format; SEQ ID NO: 17 in shNA format and combinations thereof;
when said pro-inflammatory cytokine is TNFα said nucleic acid molecules are mammalian nucleotide sequences selected from the group consisting of SEQ ID NO: 18; SEQ ID NO: 19; SEQ ID NO: 20; SEQ ID NO: 21; SEQ ID NO: 18 in shNA format; SEQ ID NO: 19 in shNA format; SEQ ID NO; 20 in shNA format; SEQ ID NO: 21 in shNA format and combinations thereof;
when said pro-inflammatory cytokine receptor is a type I IL-1β receptor said nucleic acid molecules are mammalian nucleotide sequences selected from the group consisting of SEQ ID NO: 23; SEQ ID NO: 24; SEQ ID NO: 25, SEQ ID NO: 26; SEQ ID NO: 27; SEQ ID NO: 28; SEQ ID NO: 23 in shNA format; SEQ ID NO: 24 in shNA format; SEQ ID NO: 25 in shNA format; SEQ ID NO: 26 in
shNA format; SEQ ID NO: 27 in shNA format; SEQ ID NO: 28 in shNA format and combinations thereof;
when said pro-inflammatory cytokine receptor is a type II IL-1β receptor said nucleic acid molecules are mammalian nucleotide sequences selected from the group consisting of SEQ ID NO: 29; SEQ ID NO: 30; SEQ ID NO: 31; SEQ ID NO: 32; SEQ ID NO: 33; SEQ ID NO: 34; SEQ ID NO: 29 in shNA format; SEQ ID NO: 30 in shNA format; SEQ ID NO: 31 in shNA format; SEQ ID NO: 32 in shNA format; SEQ ID NO: 33 in shNA format; SEQ ID NO: 34 in shNA format and combinations thereof;
when said pro-inflammatory cytokine receptor is a type I TNFα receptor said nucleic acid molecules are mammalian nucleotide sequences selected from the group consisting of SEQ ID NO: 35; SEQ ID NO: 36; SEQ ID NO: 37; SEQ ID NO: 38; SEQ ID NO; 39; SEQ ID NO: 40; SEQ ID NO: 35 in shNA format; SEQ ID NO: 36 in shNA format; SEQ ID NO: 37 in shNA format; SEQ ID NO: 38 in shNA format; SEQ ID NO: 39 in shNA format; SEQ ID NO: 40 in shNA format and combinations thereof;
when said pro-inflammatory cytokine receptor is a type II TNFα receptor said nucleic acid molecules are mammalian nucleotide sequences selected from the group consisting of SEQ ID NO: 41; SEQ ID NO: 42; SEQ ID NO: 43; SEQ ID NO: 44; SEQ ID NO: 45; SEQ ID NO: 46; SEQ ID NO; 41 in shNA format; SEQ ID NO: 42 in shNA format; SEQ ID NO: 43 in shNA format; SEQ ID NO: 44 in shNA format; SEQ ID NO: 45 in shNA format; SEQ ID NO: 46 in shNA format and combinations thereof.

12. Use of the kit according to claim 11 wherein said pain is acute or neuropathic.

13. Use of the kit according to claim 12, wherein said pain is sciatica.

14. Use of the kit according to claim 12, wherein said administration form is used to administer said nucleic acid molecules locally to the perispinal region of the lumbar region of a spinal cord or locally to the intrathecal space of the lumbar region of a spinal cord.

15. Use of the kit according to claim 14, wherein said administration form is selected from one or more of the group consisting of a catheter and drug pump, one or more local injections and polymer release.

## Patentansprüche

1. Verwendung von Nukleinsäuremolekülen, die Sequenzen umfassen, die die Expression von Aminosäuresequenzen unterdrücken, die für Entzündungsreaktionsproteine codieren, bei der Herstellung eines Arzneimittels zur therapeutischen Behandlung von Schmerzen durch lokale Verabreichung, wobei die Entzündungsreaktionsproteine proinflammatorische Zytokine, die aus der Gruppe bestehend aus Interleukin-1-beta ("IL-1β") und Tumornekrosefaktor-alpha ("TNFα") ausgewählt sind, oder proinflammatorische Zytokinrezeptoren sind, die aus der Gruppe bestehend aus Typ-I-IL-1β-Rezeptoren, Typ-II-IL-1β-Rezeptoren, Typ-I-TNFα-Rezeptoren und Typ-II-TNFα-Rezeptoren ausgewählt sind, **dadurch gekennzeichnet, dass**
wenn das proinflammatorische Zytokin IL-1β ist, die Nukleinsäuremoleküle Säugetier-Nukleotidsequenzen umfassen, die aus der Gruppe bestehend aus SEQ ID NR. 14, SEQ ID NR. 15, SEQ ID NR. 16, SEQ ID NR. 17, SEQ ID NR. 14 im shNA-Format, SEQ ID NR. 15 im shNA-Format, SEQ ID NR. 16 im shNA-Format, SEQ ID NR. 17 im shNA-Format und Kombinationen davon ausgewählt sind; oder wenn das proinflammatorische Zytokin TNFα ist, die Nukleinsäuremoleküle Säugetier-Nukleotidsequenzen umfassen, die aus der Gruppe bestehend aus SEQ ID NR. 18, SEQ ID NR. 19, SEQ ID NR. 20, SEQ ID NR. 21, SEQ ID NR. 18 im shNA-Format, SEQ ID NR. 19 im shNA-Format, SEQ ID NR. 20 im shNA-Format, SEQ ID NR. 21 im shNA-Format und Kombinationen davon ausgewählt sind; oder wenn der proinflammatorische Zytokinrezeptor ein Typ-I-IL-1β-Rezeptor ist, die Nukleinsäuremoleküle Säugetier-Nukleotidsequenzen umfassen, die aus der Gruppe bestehend aus SEQ ID NR. 23, SEQ ID NR. 24, SEQ ID NR. 25, SEQ ID NR. 26, SEQ ID NR. 27, SEQ ID NR. 28, SEQ ID NR. 23 im shNA-Format, SEQ ID NR. 24 im shNA-Format, SEQ ID NR. 25 im shNA-Format, SEQ ID NR. 26 im shNA-Format, SEQ ID NR. 27 im shNA-Format, SEQ ID NR. 28 im shNA-Format und Kombinationen davon ausgewählt sind;
oder wenn der proinflammatorische Zytokinrezeptor ein Typ-II-IL-1β-Rezeptor ist, die Nukleinsäuremoleküle Säugetier-Nukleotidsequenzen umfassen, die aus der Gruppe bestehend aus SEQ ID NR. 29, SEQ ID NR. 30, SEQ ID NR. 31, SEQ ID NR. 32, SEQ ID NR. 33, SEQ ID NR. 34, SEQ ID NR. 29 im shNA-Format, SEQ ID NR. 30 im shNA-Format, SEQ ID NR. 31 im shNA-Format, SEQ ID NR. 32 im shNA-Format, SEQ ID NR. 33 im shNA-Format, SEQ ID NR. 34 im shNA-Format und Kombinationen davon ausgewählt sind;
oder wenn der proinflammatorische Zytokinrezeptor ein Typ-I-TNFα-Rezeptor ist, die Nukleinsäuremoleküle Säugetier-Nukleotidsequenzen umfassen, die aus der Gruppe bestehend aus SEQ ID NR. 35, SEQ ID NR. 36, SEQ ID NR. 37, SEQ ID NR. 38, SEQ ID NR. 39, SEQ ID NR. 40, SEQ ID NR. 35 im shNA-Format, SEQ ID NR. 36 im shNA-Format, SEQ ID NR. 37 im shNA-Format, SEQ ID NR. 38 im shNA-Format, SEQ ID NR. 39 im shNA-Format, SEQ ID NR. 40 im shNA-Format und Kombinationen davon ausgewählt sind;
oder wenn der proinflammatorische Zytokinrezeptor ein Typ-II-TNFα-Rezeptor ist, die Nukleinsäuremoleküle Säugetier-Nukleotidsequenzen umfassen, die aus der Gruppe bestehend aus SEQ ID NR. 41, SEQ ID NR. 42, SEQ ID NR. 43, SEQ ID NR. 44, SEQ ID NR. 45, SEQ ID NR. 46, SEQ ID NR. 41 im shNA-Format, SEQ ID NR. 42 im shNA-Format, SEQ ID NR. 43 im shNA-Format, SEQ ID NR. 44 im shNA-Format, SEQ ID NR. 45 im shNA-Format, SEQ ID NR. 46 im shNA-Format und Kombinationen davon ausgewählt sind.

2. Verwendung nach Anspruch 1, wobei die Schmerzen akut oder neuropathisch sind.

3. Verwendung nach Anspruch 1, wobei die Schmerzen Ischiasbeschwerden sind.

4. Verwendung nach Anspruch 1, wobei die Nukleinsäuremoleküle lokal an die perispinale Region der Lendenregion eines Rückenmarks verabreicht werden sollen oder lokal an den intrathekalen Raum der Lendenregion eines Rückenmarks verabreicht werden sollen.

5. Verwendung nach Anspruch 1, wobei die Nukleinsäuremoleküle lokal von einem der Verabreichungswege verabreicht werden sollen, der aus der Gruppe bestehend aus einem Katheter und einer Medikamentenpumpe, einer oder mehreren lokalen Injektionen und Polymerfreisetzung ausgewählt ist.

6. Nukleinsäuremolekül, wobei die Nukleotidsequenz eine Säugetier-Sequenz ist, die aus der Gruppe bestehend aus SEQ ID NR. 14, SEQ ID NR. 15, SEQ ID NR. 16, SEQ ID NR. 17, SEQ ID NR. 14 im shNA-Format, SEQ ID NR. 15 im shNA-Format, SEQ ID NR. 16 im shNA-Format und SEQ ID NR. 17 im shNA-Format ausgewählt ist, und die Nukleotidsequenz eine Säugetier-Sequenz ist, die aus der Gruppe bestehend aus SEQ ID NR. 18, SEQ ID NR. 19, SEQ ID NR. 20, SEQ ID NR. 21, SEQ ID NR. 18 im shNA-Format, SEQ ID NR. 19 im shNA-Format, SEQ ID NR. 20 im shNA-Format und SEQ ID NR. 21 im shNA-Format ausgewählt ist.

7. Nukleinsäuremolekül, wobei die Nukleinsäuremoleküle Säugetier-Nukleotidsequenzen sind, die aus der Gruppe bestehend aus SEQ ID NR. 23, SEQ ID NR. 24, SEQ ID NR. 25, SEQ ID NR. 26, SEQ ID NR. 27, SEQ ID NR. 28, SEQ ID NR. 23 im shNA-Format, SEQ ID NR. 24 im shNA-Format, SEQ ID NR. 25 im shNA-Format, SEQ ID NR. 26 im shNA-Format, SEQ ID NR. 27 im shNA-Format und SEQ ID NR. 28 im shNA-Format ausgewählt sind.

8. Nukleinsäuremolekül, wobei die Nukleinsäuremoleküle Säugetier-Nukleotidsequenzen sind, die aus der Gruppe bestehend aus SEQ ID NR. 29, SEQ ID NR. 30, SEQ ID NR. 31, SEQ ID NR. 32, SEQ ID NR. 33, SEQ ID NR. 34, SEQ ID NR. 29 im shNA-Format, SEQ ID NR. 30 im shNA-Format, SEQ ID NR. 31 im shNA-Format, SEQ ID NR. 32 im shNA-Format, SEQ ID NR. 33 im shNA-Format und SEQ ID NR. 34 im shNA-Format ausgewählt sind.

9. Nukleinsäuremolekül, wobei die Nukleinsäuremoleküle Säugetier-Nukleotidsequenzen sind, die aus der Gruppe bestehend aus SEQ ID NR. 35, SEQ ID NR. 36, SEQ ID NR. 37, SEQ ID NR. 38, SEQ ID NR. 39, SEQ ID NR. 40, SEQ ID NR. 35 im shNA-Format, SEQ ID NR. 36 im shNA-Format, SEQ ID NR. 37 im shNA-Format, SEQ ID NR. 38 im shNA-Format, SEQ ID NR. 39 im shNA-Format und SEQ ID NR. 40 im shNA-Format ausgewählt sind.

10. Nukleinsäuremolekül, wobei die Nukleinsäuremoleküle Säugetier-Nukleotidsequenzen sind, die aus der Gruppe bestehend aus SEQ ID NR. 41, SEQ ID NR. 42, SEQ ID NR. 43, SEQ ID NR. 44, SEQ ID NR. 45, SEQ ID NR. 46, SEQ ID NR. 41 im shNA-Format, SEQ ID NR. 42 im shNA-Format, SEQ ID NR. 43 im shNA-Format, SEQ ID NR. 44 im shNA-Format, SEQ ID NR. 45 im shNA-Format und SEQ ID NR. 46 im shNA-Format ausgewählt sind.

11. Verwendung eines Kits, das Folgendes umfasst: Nukleinsäuremoleküle, die eine oder mehrere Sequenzen umfassen, die die Expression von Aminosäuresequenzen unterdrücken, die für Entzündungsreaktionsproteine codieren, bei der Herstellung eines Arzneimittels zur therapeutischen Behandlung von Schmerzen, und eine Verabreichungsform, die zur lokalen Abgabe der Nukleinsäuremoleküle verwendet wird, wobei die Entzündungsreaktionsproteine proinflammatorische Zytokine, die aus der Gruppe bestehend aus Interleukin-1-beta ("IL-1β") und Tumornekrosefaktor-alpha ("TNFα") ausgewählt sind, oder proinflammatorische Zytokinrezeptoren sind, die aus der Gruppe bestehend aus Typ-I-IL-1β-Rezeptoren, Typ-II-IL-1β-Rezeptoren, Typ-I-TNFα-Rezeptoren und Typ-II-TNFα-Rezeptoren ausgewählt sind, **dadurch gekennzeichnet, dass**
wenn das proinflammatorische Zytokin IL-1β ist, die Nukleinsäuremoleküle Säugetier-Nukleotidsequenzen umfassen, die aus der Gruppe bestehend aus SEQ ID NR. 14, SEQ ID NR. 15, SEQ ID NR. 16, SEQ ID NR. 17, SEQ ID NR. 14 im shNA-Format, SEQ ID NR. 15 im shNA-Format, SEQ ID NR. 16 im shNA-Format, SEQ ID NR. 17 im shNA-Format und Kombinationen davon ausgewählt sind; oder wenn das proinflammatorische Zytokin TNFα ist, die Nukleinsäuremoleküle Säugetier-Nukleotidsequenzen umfassen, die aus der Gruppe bestehend aus SEQ ID NR. 18, SEQ ID NR. 19, SEQ ID NR. 20, SEQ ID NR. 21, SEQ ID NR. 18 im shNA-Format, SEQ ID NR. 19 im shNA-Format, SEQ ID NR. 20 im shNA-Format, SEQ ID NR. 21 im shNA-Format und Kombinationen davon ausgewählt sind; oder wenn der proinflammatorische Zytokinrezeptor ein Typ-I-IL-1β-Rezeptor ist, die Nukleinsäuremoleküle Säugetier-Nukleotidsequenzen umfassen, die aus der Gruppe bestehend aus SEQ ID NR. 23, SEQ ID NR. 24, SEQ ID NR. 25, SEQ ID NR. 26, SEQ ID NR. 27, SEQ ID NR. 28, SEQ ID NR. 23 im shNA-Format, SEQ ID NR. 24 im shNA-Format, SEQ ID NR. 25 im shNA-Format, SEQ ID NR. 26 im shNA-Format, SEQ ID NR. 27 im shNA-Format, SEQ ID NR. 28 im shNA-Format und Kombinationen davon ausgewählt sind;
oder wenn der proinflammatorische Zytokinrezeptor ein Typ-II-IL-1β-Rezeptor ist, die Nukleinsäuremoleküle Säugetier-Nukleotidsequenzen umfassen, die aus der Gruppe bestehend aus SEQ ID NR. 29, SEQ ID NR. 30, SEQ ID NR. 31, SEQ ID NR. 32, SEQ ID NR. 33, SEQ ID NR. 34, SEQ ID NR. 29 im shNA-Format, SEQ ID NR. 30 im shNA-Format, SEQ ID NR. 31 im shNA-Format, SEQ ID NR. 32 im shNA-Format, SEQ ID NR. 33 im shNA-Format, SEQ ID NR. 34 im shNA-Format und Kombinationen davon ausgewählt sind;
oder wenn der proinflammatorische Zytokinrezeptor ein Typ-I-TNFα-Rezeptor ist, die Nukleinsäuremoleküle Säugetier-Nukleotidsequenzen umfassen, die aus der Gruppe bestehend aus SEQ ID NR. 35, SEQ ID NR. 36, SEQ ID NR. 37, SEQ ID NR. 38, SEQ ID NR. 39, SEQ ID NR. 40, SEQ ID NR. 35 im shNA-Format, SEQ ID NR. 36 im shNA-Format, SEQ ID NR. 37 im shNA-Format, SEQ ID NR. 38 im shNA-Format, SEQ ID NR. 39 im shNA-Format, SEQ ID NR. 40 im shNA-Format und Kombinationen davon ausgewählt sind;
oder wenn der proinflammatorische Zytokinrezeptor ein Typ-II-TNFα-Rezeptor ist, die Nukleinsäuremoleküle Säugetier-Nukleotidsequenzen umfassen, die aus der Gruppe bestehend aus SEQ ID NR. 41, SEQ ID NR. 42, SEQ ID NR. 43, SEQ ID NR. 44, SEQ ID NR. 45, SEQ ID NR. 46, SEQ ID NR. 41 im shNA-Format, SEQ ID NR. 42 im shNA-Format, SEQ ID NR. 43 im shNA-Format, SEQ ID NR. 44 im shNA-Format, SEQ ID NR. 45 im shNA-Format, SEQ ID NR. 46 im shNA-Format und Kombinationen davon ausgewählt sind.

12. Verwendung des Kits nach Anspruch 11, wobei die Schmerzen akut oder neuropathisch sind.

13. Verwendung des Kits nach Anspruch 12, wobei die Schmerzen Ischiasbeschwerden sind.

14. Verwendung des Kits nach Anspruch 12, wobei die Verabreichungsform verwendet wird, um die Nukleinsäuremoleküle lokal an die perispinale Region der Lendenregion eines Rückenmarks zu verabreichen oder lokal an den intrathekalen Raum der Lendenregion eines Rückenmarks zu verabreichen.

15. Verwendung des Kits nach Anspruch 14, wobei die Verabreichungsform aus einer oder mehreren der Gruppe bestehend aus einem Katheter und einer Medikamentenpumpe, einer oder mehreren lokalen Injektionen und Polymerfreisetzung ausgewählt ist.

## Revendications

1. Utilisation de molécules d'acide nucléique comprenant des séquences qui suppriment l'expression de séquences d'acides aminés codant pour des protéines de la réaction inflammatoire dans la fabrication d'un médicament pour le traitement thérapeutique de la douleur par administration locale, dans laquelle lesdites protéines de la réaction inflammatoire sont des cytokines pro-inflammatoires choisies dans le groupe constitué par l'interleukine-1 bêta (« IL-1β ») et le facteur de nécrose tumorale alpha (« TNFα ») ou sont des récepteurs de cytokines pro-inflammatoires choisis dans le groupe constitué par les récepteurs de l'IL-1β de type I, les récepteurs de l'IL-1β de type II, les récepteurs du TNFα de type I et les récepteurs du TNFα de type II, **caractérisée en ce que**
lorsque ladite cytokine pro-inflammatoire est l'IL-1β lesdites molécules d'acide nucléique comprennent des séquences nucléotidiques de mammifère choisies dans le groupe constitué par SEQ ID n° : 14 ; SEQ ID n° : 15 ; SEQ ID n° : 16 ; SEQ ID n° : 17 ; SEQ ID n° : 14 en format de petit acide nucléique en épingle à cheveux (shNA) ; SEQ ID n° : 15 en format shNA ; SEQ ID n° : 16 en format shNA ; SEQ ID n° : 17 en format shNA et les combinaisons de celles-ci ;
ou lorsque ladite cytokinepro-inflammatoire est le TNFα lesdites molécules d'acide nucléique sont des séquences nucléotidiques de mammifère choisies dans le groupe constitué par SEQ ID n° : 18 ; SEQ ID n° : 19 ; SEQ ID n° : 20 ; SEQ ID n° : 21 ; SEQ ID n° : 18 en format shNA ; SEQ ID n° : 19 en format shNA ; SEQ ID n° : 20 en format shNA ; SEQ ID n° : 21 en format shNAet les combinaisons de celles-ci ;
ou lorsque ledit récepteur de cytokine pro-inflammatoire est un récepteur de l'IL-1β de type I lesdites molécules d'acide nucléique sont des séquences nucléotidiques de mammifère choisies dans le groupe constitué par SEQ ID n° : 23 ; SEQ ID n° : 24 ; SEQ ID n° : 25 ; SEQ ID n° : 26 ; SEQ ID n° : : 27 ; SEQ ID n° : : 28 ; SEQ ID n° : : 23 en format shNA ; SEQ ID n° : 24 en format ShNA ; SEQ ID n° : 25 en format shNA ; SEQ ID n° : 26 en format shNA ; SEQ ID n° : 27 en format shNA ; SEQ ID n° : 28 en format shNA et les combinaisons de celles-ci ;
ou lorsque ledit récepteur de cytokine pro-inflammatoire est un récepteur de l'IL-1β de type II lesdites molécules d'acide nucléique sont des séquences nucléotidiques de mammifère choisies dans le groupe constitué par SEQ ID n° : 29 ; SEQ ID n° :30 ; SEQ ID n° :31 ; SEQ ID n° :32 ; SEQ ID n° :33 ; SEQ ID n° :34 ; SEQ ID n° :29 en format shNA ; SEQ ID n° :30 en format shNA ; SEQ ID n° :31 en format shNA ; SEQ ID n° :32 en format shNA ; SEQ ID n° :33 en format shNA ; SEQ ID n° :34 en format shNA et les combinaisons de celles-ci ;
ou lorsque ledit récepteur de cytokine pro-inflammatoire est un récepteur du TNFα de type I lesdites molécules d'acide nucléique sont des séquences nucléotidiques de mammifère choisies dans le groupe constitué par SEQ ID n° : 35 ; SEQ ID n° : 36 ; SEQ ID n° : 37 ; SEQ ID n° : 38 ; SEQ ID n° : 39 ; SEQ ID n° : 40 ; SEQ ID n° : 35 en format shNA ; SEQ ID n° : 36 en format shNA ; SEQ ID n° : 37 en format shNA ; SEQ ID n° : 38 en format shNA ; SEQ ID n° : 39 en format shNA ; SEQ ID n° : 40 en format shNA et les combinaisons de celles-ci ;
ou lorsque ledit récepteur de cytokine pro-inflammatoire est un récepteur du TNFα de type II lesdites molécules d'acide nucléique sont des séquences nucléotidiques de mammifère choisies dans le groupe constitué par SEQ ID n° : 41 ; SEQ ID n° : 42 ; SEQ ID n° : 43 ; SEQ ID n° : 44 ; SEQ ID n° : 45 ; SEQ ID n° : 46 ; SEQ ID n° : 41 en format shNA ; SEQ ID n° : 42 en format shNA ; SEQ ID n° : 43 en format shNA ; SEQ ID n° : 44 en format shNA ; SEQ ID n° : 45 en format shNA ; SEQ ID n° : 46 en format shNA et les combinaisons de celles-ci.

2. Utilisation selon la revendication 1, dans laquelle ladite douleur est aiguë ou neuropathique.

3. Utilisation selon la revendication 1, dans laquelle ladite douleur est la sciatique.

4. Utilisation selon la revendication 1, dans laquelle lesdites molécules d'acide nucléique sont destinées à être administrées localement à la région péri-rachidienne de la région lombaire d'une moelle épinière ou sont destinées à être administrées localement à l'espace intrathécal de la région lombaire d'une moelle épinière.

5. Utilisation selon la revendication 1, dans laquelle lesdites molécules d'acide nucléique sont destinées à être administrées localement à partir de l'une des voies d'administration choisies dans le groupe constitué par un cathéter et une pompe à médicament, une ou plusieurs injections locales et la libération à partir d'un polymère.

6. Molécule d'acide nucléique, dans laquelle ladite séquence nucléotidique est une séquence de mammifère choisie dans le groupe constitué par SEQ ID n° : 14 ; SEQ ID n° : 15 ; SEQ ID n° : 16 ; SEQ ID n° : 17 ; SEQ ID n° : 14 en format shNA ; SEQ ID n° : 15 en format shNA ; SEQ ID n° : 16 en format shNA ; etSEQ ID n° : 17 en format shNAet ladite séquence nucléotidique est une séquence de mammifère choisie dans le groupe constitué par SEQ ID n° : 18 ; SEQ ID n° : 19 ; SEQ ID n° : 20 ; SEQ ID n° : 21 ; SEQ ID n° : 18 en format shNA ; SEQ ID n° : 19 en format shNA ; SEQ ID n° : 20 en format shNA ; et SEQ ID n° : 21 en format shNA.

7. Molécule d'acide nucléique, dans laquelle lesdites molécules d'acide nucléique sont des séquences nucléotidiques de mammifère choisies dans le groupe constitué par SEQ ID n° : 23 ; SEQ ID n° : 24 ; SEQ ID n° : 25 ; SEQ ID n° : 26 ; SEQ ID n° : 27 ; SEQ ID n° : 28 ; SEQ ID n° : 23 en format shNA ; SEQ ID n° : 24 en format shNA ; SEQ ID n° : 25 en format shNA ; SEQ ID n° : 26 en format shNA ; SEQ ID n° : 27 en format shNA ; et SEQ ID n° : 28 en format shNA.

8. Molécule d'acide nucléique, dans laquelle lesdites molécules d'acide nucléique sont des séquences nucléotidiques de mammifère choisies dans le groupe constitué par SEQ ID n° : 29 ; SEQ ID n° : 30 ; SEQ ID n° : 31 ; SEQ ID n° : 32 ; SEQ ID n° : 33 ; SEQ ID n° : 34 ; SEQ ID n° : 29 en format shNA ; SEQ ID n° : 30 en format shNA ; SEQ ID n° : 31 en format shNA ; SEQ ID n° : 32 en format shNA ; SEQ ID n° : 33 en format shNA ; et SEQ ID n° : 34 en format shNA.

9. Molécule d'acide nucléique, dans laquelle lesdites molécules d'acide nucléique sont des séquences nucléotidiques de mammifère choisies dans le groupe constitué par SEQ ID n° : 35 ; SEQ ID n° : 36 ; SEQ ID n° : -. 37 ; SEQ ID n° : 38 ; SEQ ID n° : 39 ; SEQ ID n° : 40 ; SEQ ID n° : 35 en format shNA ; SEQ ID n° : 36 en format shNA ; SEQ ID n° : 37 en format shNA ; SEQ ID n° : 38 en format shNA ; SEQ ID n° : 39 en format shNA ; et SEQ ID n° : 40 en format shNA.

10. Molécule d'acide nucléique, dans laquelle lesdites molécules d'acide nucléique sont des séquences nucléotidiques de mammifère choisies dans le groupe constitué par SEQ ID n° : 41 ; SEQ ID n° : 42 ; SEQ ID n° : 43 ; SEQ ID n° : 44 ; SEQ ID n° : 45 ; SEQ ID n° : 46 ; SEQ ID n° : 41 en format shNA ; SEQ ID n° : 42 en format shNA ; SEQ ID n° : 43 en format shNA ; SEQ ID n° : 44 en format shNA ; SEQ ID n° : 45 en format shNA ; et SEQ ID n° : 46 en format shNA.

11. Utilisation d'une trousse comprenant : des molécules d'acide nucléique comprenant une ou plusieurs séquences qui suppriment l'expression de séquences d'acides aminés codant pour des protéines de la réaction inflammatoire dans la fabrication d'un médicament pour le traitement thérapeutique de la douleur et d'une forme d'administration utilisée pour administrer localement lesdites molécules d'acide nucléique, dans laquelle lesdites protéines de la réaction inflammatoire sont des cytokines pro-inflammatoires choisies dans le groupe constitué par l'interleukine-1 bêta (« IL-1β ») et le facteur de nécrose tumorale alpha (« TNFα ») ou sont des récepteurs de cytokines pro-inflammatoires choisis dans le groupe constitué par les récepteurs de l'IL-1β de type I, les récepteurs de l'IL-1β de type II, les récepteurs du TNFα de type I et les récepteurs du TNFα de type II, **caractérisée en ce que**
lorsque ladite cytokine pro-inflammatoire est l'IL-1β lesdites molécules d'acide nucléique comprennent des séquences nucléotidiques de mammifère choisies dans le groupe constitué par SEQ ID n° : 14 ; SEQ ID n° : 15 ; SEQ ID n° : 16 ; SEQ ID n° : 17 ; SEQ ID n° : 14 en format shNA ; SEQ ID n° : 15 en format shNA ; SEQ ID n° : 16 en format shNA ; SEQ ID n° : 17 en format shNA et les combinaisons de celles-ci ;
ou lorsque ladite cytokinepro-inflammatoire est le TNFα lesdites molécules d'acide nucléique sont des séquences nucléotidiques de mammifère choisies dans le groupe constitué par SEQ ID n° : 18 ; SEQ ID n° : 19 ; SEQ ID n° : 20 ; SEQ ID n° : 21 ; SEQ ID n° : 18 en format shNA ; SEQ ID n° : 19 en format shNA ; SEQ ID n° : 20 en format shNA ; SEQ ID n° : 21 en format shNA et les combinaisons de celles-ci;
ou lorsque ledit récepteur de cytokine pro-inflammatoire est un récepteur de l'IL-1β de type I lesdites molécules d'acide nucléique sont des séquences nucléotidiques de mammifère choisies dans le groupe constitué par SEQ ID n° : 23 ; SEQ ID n° : 24 ; SEQ ID n° : 25 ; SEQ ID n° : 26 ; SEQ ID n° : 27 ; SEQ ID n° : 28 ; SEQ ID n° : 23 en format shNA ; SEQ ID n° : 24 en format shNA ; SEQ ID n° : 25 en format shNA ; SEQ ID n° : 26 en format shNA ; SEQ ID n° : 27 en format shNA ; SEQ ID n° : 28 en format shNA et les combinaisons de celles-ci ;
ou lorsque ledit récepteur de cytokine pro-inflammatoire est un récepteur de l'IL-1β de type II lesdites molécules d'acide nucléique sont des séquences nucléotidiques de mammifère choisies dans le groupe constitué par SEQ ID n° : 29 ; SEQ ID n° : 30 ; SEQ ID n° : 31 ; SEQ ID n° : 32 ; SEQ ID n° : 33 ; SEQ ID n° : 34 ; SEQ ID n° : 29 en format shNA ; SEQ ID n° : 30 en format shNA ; SEQ ID n° : 31 en format shNA ; SEQ ID n° : 32 en format shNA ; SEQ ID n° : 33 en format shNA ; SEQ ID n° : 34 en format shNA et les combinaisons de celles-ci;
ou lorsque ledit récepteur de cytokine pro-inflammatoire est un récepteur du TNFα de type I lesdites molécules d'acide nucléique sont des séquences nucléotidiques de mammifère choisies dans le groupe constitué par SEQ ID n° : 35 ; SEQ ID n° : 36 ; SEQ ID n° : 37 ; SEQ ID n° : 38 ; SEQ ID n° : : 39 ; SEQ ID n° : : 40 ; SEQ ID n° : 35 en format shNA ; SEQ ID n° : 36 en format shNA ; SEQ ID n° : 37 en format shNA ; SEQ ID n° : 38 en format shNA ; SEQ ID n° : 39 en format shNA ; SEQ ID n° : 40 en format shNA et les combinaisons de celles-ci;
ou lorsque ledit récepteur de cytokine pro-inflammatoire est un récepteur du TNFα de type II lesdites molécules d'acide nucléique sont des séquences nucléotidiques de mammifère choisies dans le groupe constitué par SEQ ID n° : 41 ; SEQ ID n° : 42 ; SEQ ID n° : 43 ; SEQ ID n° : 44 ; SEQ ID n° : : 45 ; SEQ ID n° : : 46 ; SEQ ID n° : 41 en format shNA ; SEQ ID n° : 42 en format shNA ; SEQ ID n° : 43 en format shNA ; SEQ ID n° : 44 en format shNA ; SEQ ID n° : 45 en format shNA ; SEQ ID n° : 46 en format shNA et les combinaisons de celles-ci.

12. Utilisation de la trousse selon la revendication 11, dans laquelle ladite douleur est aiguë ou neuropathique.

13. Utilisation de la trousse selon la revendication 12, dans laquelle ladite douleur est la sciatique.

14. Utilisation de la trousse selon la revendication 12, dans laquelle ladite forme d'administration est utilisée pour administrer lesdites molécules d'acide nucléique localement à la région péri-rachidienne de la région lombaire d'une moelle épinière ou localement à l'espace intrathécal de la région lombaire d'une moelle épinière.

15. Utilisation de la trousse selon la revendication 14, dans laquelle ladite forme d'administration est choisie parmi une ou plusieurs voies du groupe constitué par un cathéter et une pompe à médicament, une ou plusieurs injections locales et la libération à partir d'un polymère.
